# FASCICULE DE BREVET EUROPEEN

(11) **EP 0 861 247 B1**
(45) Date de publication et mention de la délivrance du brevet: **07.10.2009**
(21) Numéro de dépôt: 96938266.2
(22) Date de dépôt: 07.11.1996
(51) Int. Cl.: C07D 405/06, C07D 405/14, A61K 31/415, A61K 31/36, C07D 409/14

(54) **NOUVEAUX DERIVES D'IMIDAZOLE N-BENZYLDIOXOL, LEUR PROCEDE DE PREPARATION, INTERMEDIAIRES OBTENUS, COMPOSITIONS PHARMACEUTIQUES ET LEUR UTILISATION COMME ANTAGONISTES D'ENDOTHELINE**
NEUE N-BENZYLDIOXOL-IMIDAZOLDERIVATE, VERFAHREN ZU IHRER HERSTELLUNG, ERHALTENE ZWISCHENPRODUKTE, PHARMAZEUTISCHE ZUSAMMENSETZUNGEN UND IHRE VERWENDUNG ALS ENDOTHELIN-ANTAGONISTEN
NOVEL IMIDAZOLE N-BENZYLDIOXOL DERIVATIVES, METHOD FOR PREPARING SAME, RESULTING INTERMEDIATES, PHARMACEUTICAL COMPOSITIONS AND USE OF SAID DERIVATIVES AS ENDOTHELIN ANTAGONISTS

(30) Priorité: 08.11.1995 FR 9513189
(43) Date de publication de la demande: 02.09.1998
(73) Titulaire: Aventis Pharma S.A., 92160 Antony (FR)
(72) Inventeur: HECKMANN, Bertrand, F-94230 Cachan (FR); JOUQUEY, Simone, F-75020 Paris (FR); VEVERT, Jean-Paul, F-93500 Pantin (FR); ZHANG, Jidong, F-75013 Paris (FR)
(86) Numéro de dépôt international: PCT/FR1996/001749
(87) Numéro de publication internationale: WO 1997/017339

(56) Documents cités:
- WO-A-94/14434
- WO-A-96/04276

## Description

L'invention concerne de nouveaux dérivés d'imidazole N-benzyldioxole, leur procédé de préparation, les nouveaux intermédiaires obtenus, leur application à titre de médicaments, les compositions pharmaceutiques les renfermant et la nouvelle utilisation de tels dérivés de l'imidazole.

Le document WO9604276 décrit des dérivés de l'imidazole.

L'invention a pour objet les produits sélectionnés dans le groupe consistant en les produits dont les noms suivent ;
- acide 4-((4-(carboxymèthylene) cyclohexyl) thio)-1-((6-chloro-1,3-benzodioxol-5-yl) méthyl)-2-propyl-1H-imidazole-5-carboxylique,
- acide 4-((5-carboxypentyl) thio)-1-((6-chloro-1,3-benzodioxol-5-yl) méthyl)-2-(1,3-dioxolan-2-yl)-1H-imidazole-5-carboxylique,
- acide 4-((4-(carboxyméthyl) cyclohexyl) thio)-1-((6-chloro-1,3-benzodioxol-5-yl) méthyl)-2-propyl-1H-imidazole-5-carboxylique,
- acide 1-((6-chloro 1,3-benzodioxol-5-yl) méthyl) 4-((4-carboxycyclohexyl) thio) 2-propyl 1H-imidazole 5-carboxylique,
- acide 4(((4-carboxycyclohexyl) méthyl) thio) 1-((6-chloro 1,3-benzadioxol-5-yl) méthyl) 2-propyl 1H-imidazole 5-carboxylique,
- acide trans 4-((4-(carboxyméthyl) cyclohexyl)) thio) 1-((6-chloro 1,3-benzodioxol-5-yl)méthyl) 2-(1,3-dioxolan-2-yl) 1H-imidazole 5-carboxylique,
- acide 4-((4-(carboxyméthyl) cyclohexyl) thio) 1-((6-chloro 1,3-benzodioxol-5-yl) méthyl) 2-(2-furanyl) 1H-imidazole 5-carboxylique,
- acide trans 4-((4-(carboxyméthyl) cyclohexyl) thio) 1-((6-chloro 1,3-benzodiaxol-5-yl) méthyl) 2-cyclopentyl 1H-imidazole 5-carboxylique,
- acide 4-((4-carboxycyclohexyl)thio) 1-((6-chloro 1,3-benzodioxol-5-yl) méthyl) 2-(2-furanyl) 1H-imidazole 5-carboxylique,
- sel de disodium de l'acide 4-((4-(carboxymèthyl) cyclohexyl) thio) 1-((6-chloro 1,3-benzodioxol-5-yl) méthyl) 2-propyl 1H-imidazole 5-carboxylique,
- sel de dipotassium de l'acide 4-((4-(carboxyméthyl) cyclohexyl) thio) 1-((6-chloro 1,3-benzodioxol-5-yl) méthyl) 2-propyl 1H-imidazole 5-carboxylique,

Le ou les radicaux carboxy des produits de l'invention peuvent être salifiés ou estérifiés par les groupements divers connus de l'homme du métier parmi lesquels on peut citer, par exemple :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaine, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle.

Les sels d'addition avec les acides minéraux ou organiques des produits de l'invention peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

On peut rappeler que la stéréoisomérie peut être définie dans son sens large comme l'isomérie de composés ayant mêmes formules développées, mais dont les différents groupes sont disposés différemment dans l'espace, tels que notamment dans les formes bateau et chaise du cyclohexane et des cyclohexanes monosubstituée dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomères est utilisé dans la présente demande dans son sens le plus large et concerne donc l'ensemble des composés indiqués ci-dessus.

Pour une meilleure compréhension de l'invention, on décrit ci-après un procédé de préparation des produits de l'invention, caractérisé en ce que :
soit l'on soumet un composé de formule (II) : 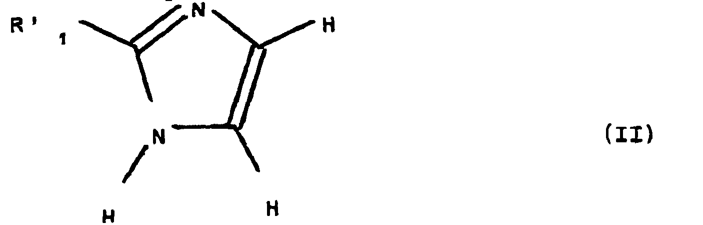 dans laquelle R'₁ représente un radical alkyle, éventuellement substitué par un ou plusieurs radicaux hydroxyle au alkoxy, les radicaux alkyle et alkoxy étant linéaires ou ramifiés et renfermant au plus 6 atomes de carbone, ou un radical cyclique saturé ou insaturé constitué de 3 à 7 chaînons, et renfermant éventuel- lement un ou plusieurs hétéroatomes identiques ou différents choisis parmi les atomes d'oxygène, de soufre et d'azote, dans laquelle les éventuelles fonctions réactives sont éventuellement protégées par des groupements protecteurs, à une réaction avec un composé de formule (III) : 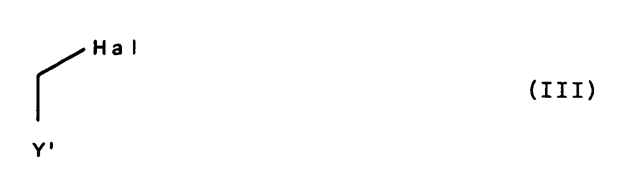 dans laquelle Hal représente un atome d'halogène, et
   Y' représente le radical phényle substitué par un radical dioxol et éventuellement substitué par un autre substituant choisi parmi les atomes d'halogène et les radicaux alcoxy et alkyle renfermant au plus 4 atomes de carbone, dans laquelle les éventuelles fonctions réactives sont éventuellement protégées par des groupements protecteurs,
   pour obtenir le produit de formule (IV) : 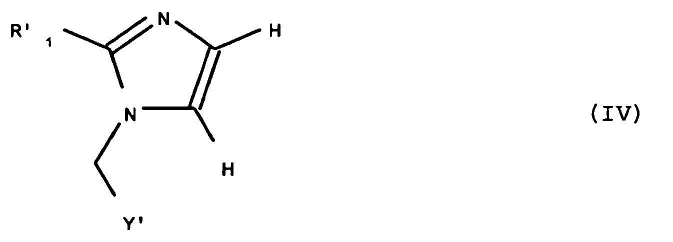 dans laquelle R'₁ et Y' ont les significations indiquées ci-dessus, produit de formule (IV) que l'on peut soumettre à une réaction d'halogénation, pour obtenir le produit de formule (V) : 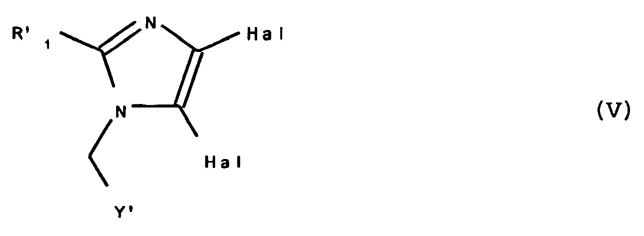 dans laquelle R'₁, Hal et Y' ont les significations indiquées ci-dessus,
soit l'on soumet un composé de formule (VI) : 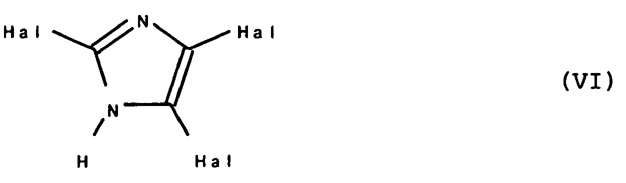 dans laquelle Hal a la signification indiquée ci-dessus,
   soit à une réaction avec le composé de formule (III) telle que définie ci-dessus, soit à l'action d'un groupement protecteur P, pour obtenir un produit de formule (VII) : 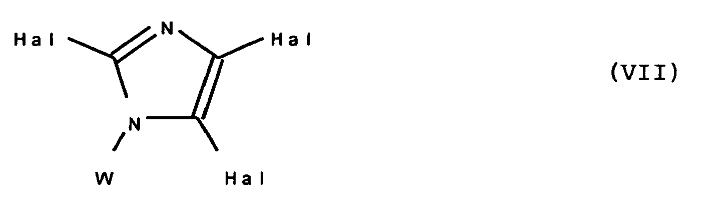 dans laquelle Hal a la signification indiquée ci-dessus et W représente soit -CH₂-Y' avec Y' tel que défini ci-dessus, soit P qui représente un groupement protecteur de l'atome d'azote, produit de formule (VII) que l'on soumet à une réaction d'échange halogène-métal puis à une réaction avec le diméthylformamide ou avec un électrophile de formule (VIIIₐ) ou (VIII_{b}) :

   L₁-CHO (VIIIₐ)

   L₁-CO-Cl (VIII_{b})

   dans lesquelles L₁ représente un radical alkyle, linéaire ou ramifié, renfermant au plus 6 atomes de carbone et éventuellement substitué par un radical alcoxy ou hydroxyle protégé, pour obtenir un produit de formule (IX) : 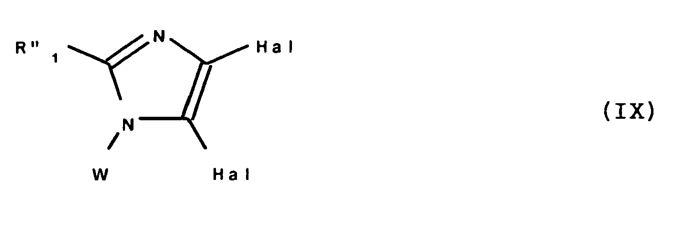 dans laquelle Hal et W ont les significations indiquées ci-dessus, R"₁ représente un radical alkyl-carbonyle, formyle ou hydroxyalkyle dans lesquels le radical alkyle a la signification indiquée ci-dessus et dans lesquels les éventuelles fonctions réactives sont éventuellement protégées par des groupements protecteurs, produits de formules (V) et (IX) que l'on peut soumettre à une réaction d'échange halogène-métal sur l'un des atomes d'halogène puis à une réaction avec CO₂ ou DMF ou un composé électrophile de formule (X) : 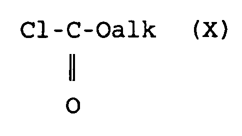 dans lequel alk représente un radical alkyle renfermant au plus 4 atomes de carbone,
   pour obtenir le composé de formule (XI) : 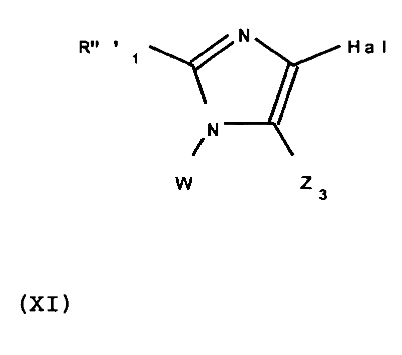 dans laquelle R"'₁ représente R'₁ ou R"₁ tels que définis ci-dessus, Hal et W ont les significations indiquées ci-dessus et Z₃ représente le radical carboxy, libre ou estérifié ou le radical formyle,
   composé de formule (XI) que l'on peut soumettre à une réaction avec un composé de formule (XII) :

   A'-S-M (XII)

   dans laquelle S représente un atome de soufre, M représente un métal tel que sodium, potassium ou cuivre et A' représente A tel que défini ci-dessus, dans lequel les éventuelles fonctions réactives sont éventuellement protégées par des groupements protecteurs pour obtenir le composé de formule (XIII) : 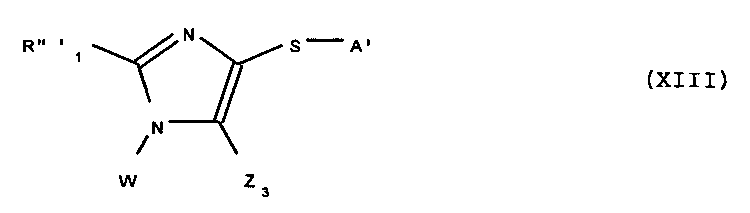 dans laquelle R"'₁, Y', A' , Z₃ et W ont les significations indiquées ci-dessus, produit de formule (XIII) que lorsque Z₃ représente le radical formyle, l'on peut soumettre à une réaction d'oxydation pour obtenir le produit de formule (XIV) : 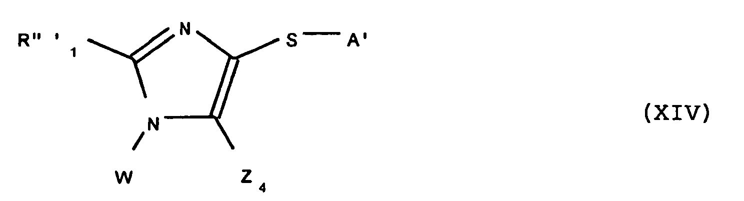 dans laquelle R"'₁, A' et W ont les significations indiquées ci-dessus, et Z₄ représente le radical cyano ou le radical carboxy libre ou estérifié par un radical alkyle linéaire ou ramifié renfermant au plus 6 atomes de carbone, produits de formule (XIII) ou (XIV) que, dans le cas où W représente P tel que défini ci-dessus et après libération de la fonction amine bloquée par P tel que défini ci-dessus, l'on fait réagir avec le composé de formule (III) telle que définie ci-dessus, pour obtenir un produit de formule (XV) : 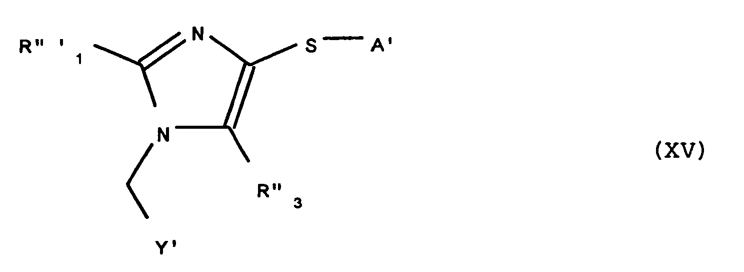 dans laquelle R"'₁, A' et Y' ont les significations indiquées ci-dessus, et R"₃ représente Z₃ ou Z₄ tels que définis ci-dessus,
soit l'on soumet un composé de formule (XVI) : 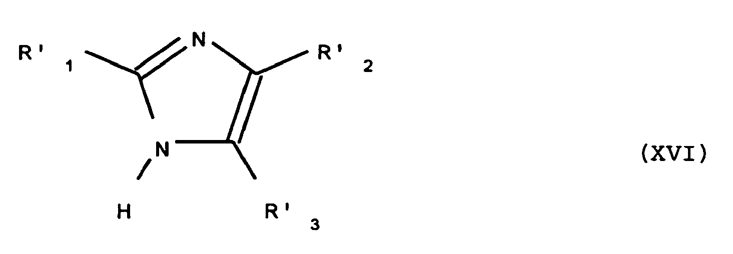 dans laquelle R'₁ a la signification indiquée ci-dessus et R'₂ et R'₃ identiques ou différents, représentent ou portent tous deux une fonction acide ou isostère d'acide
   dans lesquelles les éventuelles fonctions réactives sont éventuellement protégées par des groupements protecteurs, à une réaction avec le composé de formule (III) telle que définie ci-dessus, pour obtenir un produit de formule (I₁): 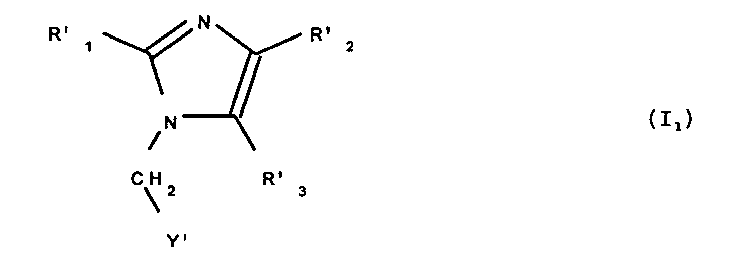 dans laquelle R'₁, R'₂, R'₃ et Y' ont les définitions indiquées ci-dessus,
   soit l'on soumet un composé de formule (XVII) : 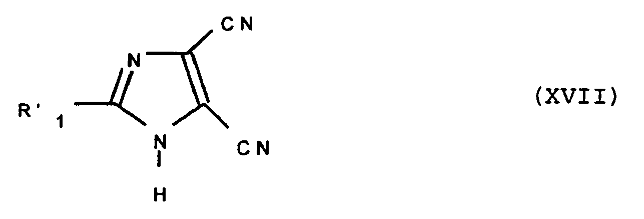 dans laquelle R'₁ a la signification indiquée précédemment, à une réaction d'hydrolyse pour obtenir le produit de formule (XVIII) _{:} 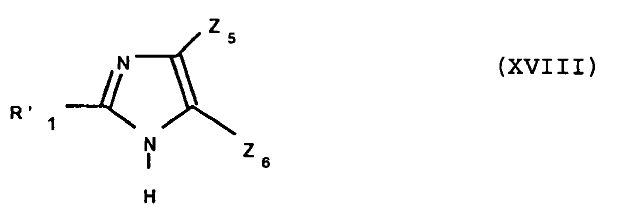 dans laquelle R'₁ a la signification indiquée précédemment et l'un ou les deux de Z₅ et Z₆ représentent un radical carboxy libre ou estérifié et le cas échéant, l'autre de Z₅ et Z₆ conservant la valeur CN, produit de formule (XVIII) que l'on fait réagir avec le composé de formule (III) telle que définie ci-dessus pour obtenir le produit de formule (XIX) : 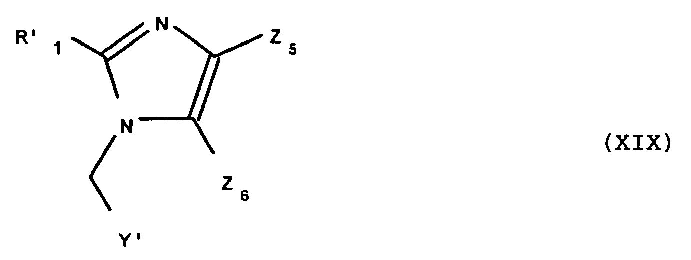 dans laquelle R'₁, Y', Z₅ et Z₆ ont les significations indiquées précédemment,
soit l'on soumet un composé de formule (XX) :

   Y'-CHO (XX)

   dans laquelle Y' a la signification indiquée ci-dessus, à un composé de formule (XXI) : 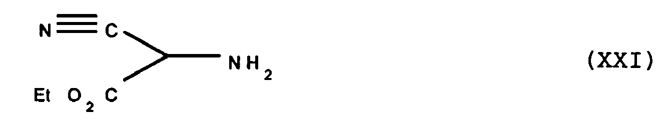 pour obtenir après réduction de l'imine intermédiaire un produit de formule (XXII) : 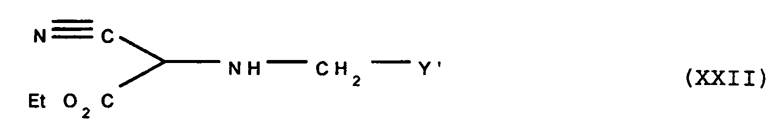 dans laquelle Y' a la signification indiquée ci-dessus, que l'on soumet à un composé de formule (XXIII) : 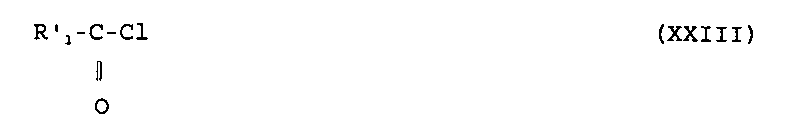 dans laquelle R'₁ a la signification indiquée ci-dessus, pour obtenir le produit de formule (XXIV) : 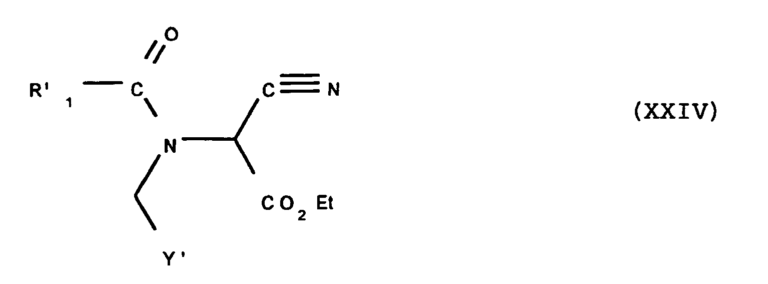 dans laquelle Y' et R'₁ ont les significations indiquées ci-dessus, que l'on soumet à un composé de formule (XXV) :

   A'-S-H (XXV)

   dans laquelle A' a la signification indiquée ci-dessus pour obtenir le produit de formule (XXVI) : 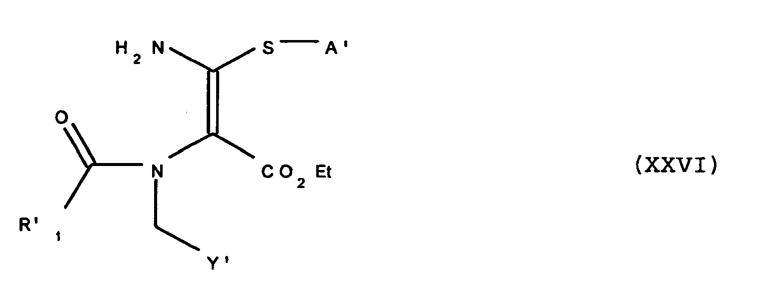 dans laquelle Y', R'₁ et A' ont les significations indiquées ci-dessus,
   qui est cyclisé en produit de formule (I₂) : 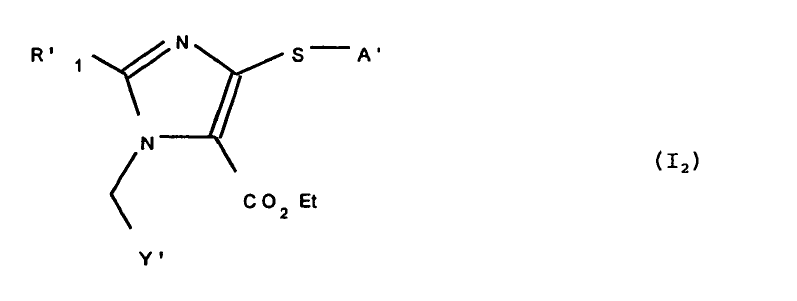 dans laquelle Y', R'₁ et A' ont les significations indiquées ci-dessus,
   soit l'on soumet un composé de formule (XXI) : 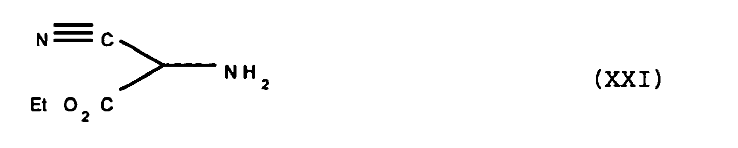 à l'action d'un composé de formule (XXIII) : 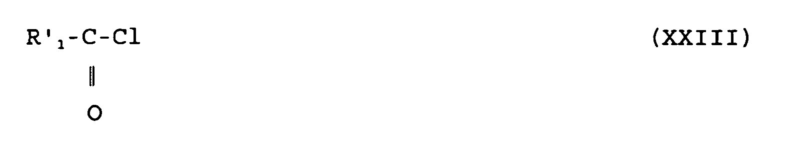 dans laquelle R'₁ a la signification indiquée ci-dessus pour obtenir un composé de formule (XXIVa) : 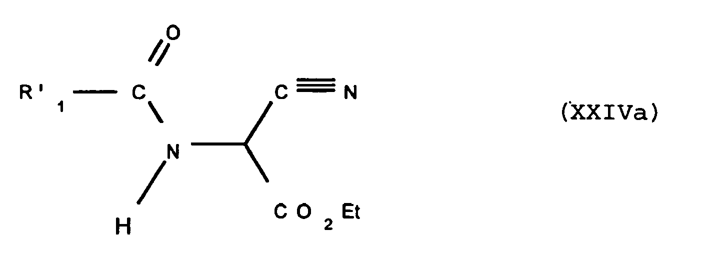 dans laquelle R'₁ a la signification indiquée ci-dessus, que l'on soumet à l'action d'un composé de formule (XXV) :

   A'-S-H (XXV)

   dans laquelle A' a la signification indiqué ci-dessus pour obtenir un produit de formule (XXVIa) : 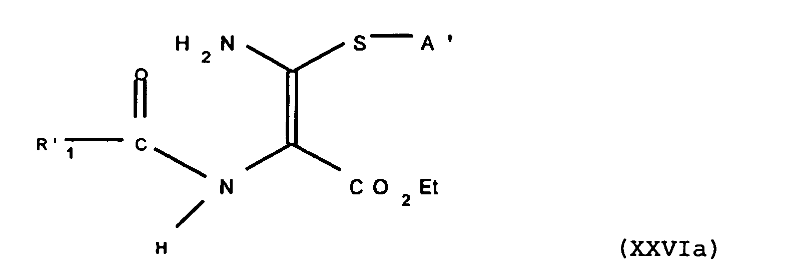 dans laquelle R'₁ et A' ont les significations indiquées précédemment, que l'on cyclise en produit de formule (XXVIb): 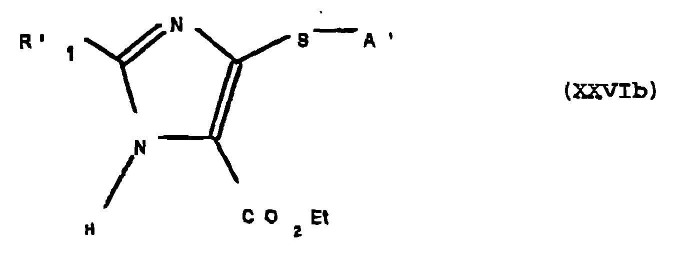 dans laquelle R'₁ et A' ont la signification indiquée précédemment, que l'on soumet à l'action d'un produit de formule (III) : 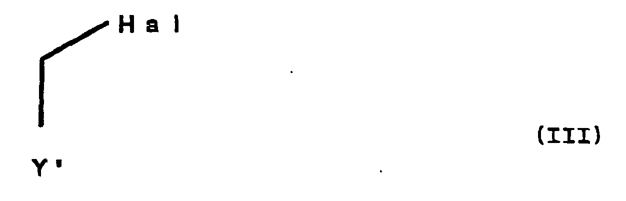 dans laquelle Hal et Y' ont la signification indiquée ci-dessus pour obtenir un produit de formule (I₂) telle que définie ci-dessus, produits de formules (IX), (XI), (XIII), (XIV), (XV), (XIX), (I₁) et (I₂) qui peuvent être des produits de l'invention et que, pour obtenir des ou d'autres produits de l'invention, l'on peut soumettre, si désiré et si nécessaire, à l'une ou plusieurs des réactions de transformations suivantes, dans un ordre quelconque :
   a) une réaction d'estérification de fonction acide,
   b) une réaction de saponification de fonction ester en fonction acide,
   c) une réaction de transformation de fonction ester en fonction formyle,
   d) une réaction de transformation de la fonction cyano en fonction acide,
   e) une réaction d'oxydation de groupement alkylthio en sulfoxyde ou sulfone correspondant,
   f) une réaction de réduction de la fonction carboxy libre ou estérifié en fonction alcool,
   g) une réaction de transformation de fonction alcoxy en fonction hydroxyle, ou encore de fonction hydroxyle en fonction alcoxy,
   h) une réaction d'oxydation de fonction alcool en fonction aldéhyde, acide ou cétone,
   i) une réaction de transformation du radical formyle en radical carbamoyle,
   j) une réaction de transformation du radical carbamoyle en radical nitrile,
   k) une réaction de transformation de radical nitrile en tétrazolyle,
   l) une réaction de transformation d'une fonction halogénée en fonction formyle ou carboxy estérifié,
   m) une réaction de transformation d'un radical formyle en fonction CH₂-CO₂alk ou CH=CH-CO₂alk dans laquelle alk représente un radical alkyle renfermant de 1 à 4 atomes de carbone, puis le cas échéant, transformation en acide correspondant,
   n) une réaction de transformation d'un radical formyle en radical CH=CH-A' puis le cas échéant en radical 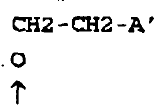
   o) une réaction d'oxydation du radical S-alk en S-alk puis transformation en fonction SH et le cas échéant en S-A' dans lequel alk et A' ont la signification indiquée précédemment,
   p) une réaction d'élimination des groupements protecteurs que peuvent porter les fonctions réactives protégées,
   q) une réaction de salification par un acide minéral ou organique ou par une base pour obtenir le sel correspondant,
   r) une réaction de dédoublement des formes racémiques en produits dédoublés,
      lesdits produits de l'invention ainsi obtenus étant sous toutes les formes isomères possibles racémiques, énantiomères et diastéréoisomères.

On peut noter que de telles réactions de transformation de substituants en d'autres substituants peuvent également être effectuées sur les produits de départ ainsi que sur les intermédiaires tels que définis ci-dessus avant de poursuivre la synthèse selon les réactions indiquées dans le procédé décrit ci-dessus.

Dans des conditions préférentielles de mise en oeuvre de l'invention, le procédé décrit ci-dessus peut-être réalisé de la façon suivante :

Dans le produit de formule (III), l'atome d'halogène représente de préférence un atome de brome mais peut également représenter un atome de chlore ou d'iode. La réaction de condensation des imidazoles de formules telles que définies ci-dessus (II), (VI), (XVI), (XIII) et (XIV) (dans le cas des produits de formules (XIII) et (XIV), lorsque W représente P et après déprotection de l'atome d'azote), avec le composé de formule (III) telle que définie ci-dessus, pour obtenir respectivement les produits de formules (IV), (VII) lorsque W représente Y', (XV) et (I₁) telles que définies ci-dessus peut être réalisée dans un solvant tel que par exemple le diméthylformamide ou encore le diméthylacétamide, le tétrahydrofuranne, le diméthoxyéthane ou le diméthylsulfoxyde au reflux du solvant ou à la température ambiante, de préférence sous agitation ; la réaction est réalisée de préférence en présence d'une base telle que par exemple l'hydrure de sodium ou de potassium ou encore du carbonate de sodium ou de potassium, du méthylate ou éthylate ou tert-butylate de sodium ou de potassium.

La réaction d'halogénation du composé de formule (IV) telle que définie ci-dessus en composé de formule (V) telle que définie ci-dessus, peut être réalisée dans les conditions usuelles de l'homme du métier et notamment par bromation à l'aide de NBS dans CH₂Cl₂ ou encore Br₂ dans l'acide acétique.

Les composés de formules (V), (VII) et (IX) telles que définies ci-dessus peuvent être soumis à une réaction d'échange halogène métal sur l'atome d'halogène par réaction avec un composé organo métallique tel que le nBuli ou le bromure d'éthyle magnésien dans un solvant tel que le tétrahydrofuranne à une température d'environ -78°C pour le Buli et température ambiante pour le bromure d'éthyle magnésien.

La réaction de carboxylation par CO₂ et de formylation par le diméthylformamide des composés de formules (V) ou (IX) en composé de formule (XI) peut être réalisée selon les conditions usuelles de l'homme de métier soit par exemple dans le tétrahydrofuranne à température ambiante.

L₁ représente un radical alkyle de telle manière que R_{1"} représente les valeurs correspondantes choisies parmi les valeurs de R₁ telles que définies ci-dessus dans lesquelles les éventuelles fonctions réactives sont éventuellement protégées par des groupements protecteurs.

La réaction du composé de formule (V) ou (IX) telles que définies ci-dessus avec le composé de formule (X), telle que définie ci-dessus, pour obtenir le composé correspondant de formule respective (XI) telle que définie ci-dessus peut être réalisée d'une manière identique en utilisant le bromure d'éthyle magnésien comme agent de métallation dans le tétrahydrofuranne à température ambiante.

La réaction du composé de formule (VII) avec les composés de formules (VIIIa) ou (VIIIb) peut être réalisée selon les conditions usuelles de l'homme de métier soit par exemple dans le tétrahydrofuranne à température ambiante.

La fonction amine des composés de formules (XIII) et (XIV) telles que définies ci-dessus, protégée par P tel que défini ci-dessus, peut être libérée dans les conditions usuelles connues de l'homme du métier et notamment lorsque P représente le radical -CH₂-O-(CH₂)₂-Si(CH₃)₃, l'atome d'hydrogène peut être libéré dans de l'acide trifluoroacétique ou encore en présence d'ion fluorure.

La réaction de saponification peut être réalisée selon les méthodes usuelles connues de l'homme du métier, telles que par exemple dans un solvant tel que le méthanol ou l'éthanol, le dioxane ou le diméthoxyéthane, en présence de soude ou de potasse ou encore de carbonate de cesium.

Les réactions de réduction ou oxydation du produit de formule (XIII) en produit de formules (XIV) peuvent être réalisées selon les méthodes usuelles connues de l'homme du métier.

Dans les réactions décrites ci-dessus, on peut opérer de la façon suivante :
- la réaction du composé de formule (xx) avec le composé de formule (XXI) pour obtenir le composé de formule (XXII) peut être réalisée dans un solvant tel que par exemple le chlorure de méthylène en présence d'un catalyseur acide tel que par exemple l'amberlist puis par réduction par exemple par le borohydrure de sodium dans de l'acide acétique et du chlorure de méthylène,
- la réaction du composé de formule (XXII) ou (XXI) avec le composé de formule (XXIII) pour obtenir respectivement le composé de formule (XXIV) ou (XXIVa) peut être réalisée dans un solvant tel que par exemple le tétrahydrofuranne ou le chlorure de méthylène, en présence d'une base telle que la pyridine ou encore la triéthylamine ou le carbonate de sodium ou de potassium,
- la réaction du composé de formule (XXIV) ou (XXIVa) avec le composé de formule (XXV) pour obtenir respectivement le composé de formule (XXVI) ou (XXVIa) peut être réalisée dans un alcool tel que le méthanol ou l'éthanol en présence d'une base telle que la triéthylamine ou la pyridine.
- la cyclisation du composé de formule (XXVI) ou (XXVIa) respectivement en produit de formule (I₂) ou (XXVIb) peut être réalisée en présence d'un anhydride tel que par exemple l'anhydride propane phosphonique dans de l'acétate d'éthyle.
- la réaction du composé de formule (XXVIa) avec le composé de formule (III) peut être réalisée de façon identique à celle indiquée ci-dessus pour la réaction de condensation du produit de formule (III) et des imidazole de formules (II), (VI), (XVI), (XIII) et (XIV).

Selon les valeurs de R'₁, R"₁, R"'₁, R'₂, R'₃, R"₃, les produits de formules (IX), (XI), (XIII), (XIV), (XV), (XIX), (I₁) et (I₂) constituent ou non des produits de l'invention et peuvent donner des produits de l'invention, ou être transformés en d'autres produits de l'invention en étant soumis à une ou plusieurs des réactions a) à r) indiquées ci-dessus.

Ainsi les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyle, trityle, benzyle, tert-butoxycarbonyle, phtalimido ou d'autres radicaux connus dans la chimie des peptides,
- les groupements acyles tel que le groupement formyle peuvent être protégés par exemple sous forme de cétals ou de thiocétals cycliques ou non cycliques tels que le diméthyl ou diéthylcétal ou l'éthylène dioxycétal, ou le diéthylthiocétal ou l'éthylènedithiocétal,
- les fonctions acide des produits décrits ci-dessus peuvent être, si désiré, amidifiées par une amine primaire ou secondaire par exemple dans du chlorure de méthylène en présence, par exemple, de chlorhydrate de 1-éthyl-3-(diméthylaminopropyl) carbodiimide à la température ambiante :
- les fonctions acide peuvent être protégées par exemple sous forme d'esters formés avec les esters facilement clivables tels que les esters benzyliques ou ter butyliques ou des esters connus dans la chimie des peptides.

Les réactions auxquelles les produits de formules (IX), (XI), (XIII), (XIV), (XV), (XIX), (I₁) et (I₂) telles que définies ci-dessus peuvent être soumis, si désiré ou si nécessaire, peuvent être réalisées, par exemple, comme indiqué ci-après.
a) Les produits décrits ci-dessus peuvent, si désiré, faire l'objet, sur les éventuelles fonctions carboxy, de réactions d'estérification qui peuvent être réalisées selon les méthodes usuelles connues de l'homme du métier.
b) Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.
c) la réaction de transformation de la fonction ester 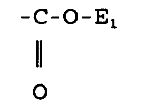 dans laquelle E₁ peut représenter un radical alkyle éventuellement substitué et éventuellement protégé, en radical formyle peut être réalisée ainsi qu'il est décrit dans la partie expérimentale, ou selon les méthodes usuelles connues de l'homme du métier, notamment par saponification de l'ester en acide, transformé alors en chlorure d'acide par exemple par action du chlorure de thionyle, et réduit ensuite par exemple par hydrogénation sur palladium.
d) Les éventuelles fonctions cyano des produits décrits ci-dessus peuvent être, si désiré, transformées en fonction acide dans les conditions usuelles connues de l'homme du métier par exemple par une double hydrolyse réalisée en milieu acide tel que par exemple dans un mélange d'acide sulfurique, d'acide acétique glacial et d'eau, ces trois composés étant de préférence en proportions égales, ou encore dans un mélange de soude, d'éthanol et d'eau au reflux.
e) Les éventuels groupements alkylthio des produits décrits ci-dessus peuvent être, si désiré, transformés en les fonctions sulfoxyde ou sulfone correspondantes dans les conditions usuelles connues de l'homme du métier telles que par exemple par les peracides comme par exemple l'acide peracétique ou l'acide métachloroperbenzoique ou encore par l'ozone, l'oxone, le périodate de sodium dans un solvant tel que par exemple le chlorure de méthylène ou le dioxanne à la température ambiante.
   L'obtention de la fonction sulfoxyde peut être favorisée par un mélange équimolaire du produit renfermant un groupement alkylthio et du réactif tel que notamment un peracide.
   L'obtention de la fonction sulfone peut être favorisée par un mélange du produit renfermant un groupement alkylthio avec un excès du réactif tel que notamment un peracide.
f) Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme du métier et notamment par de l'hydrure de lithium et d'aluminium dans un solvant tel que par exemple le tétrahydrofuranne ou encore le dioxane ou l'éther éthylique.
   Les éventuelles fonctions carboxy libre des produits décrits ci-dessus peuvent être, si désiré, réduites en fonction alcool notamment par de l'hydrure de bore.
g) Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide trifluoro acétique au reflux.
h) Les éventuelles fonctions alcool des produits décrits ci-dessus peuvent être, si désiré, transformées en fonction aldéhyde ou acide par oxydation dans les conditions usuelles connues de l'homme du métier telles que par exemple par action de l'oxyde de manganèse pour obtenir les aldéhydes ou du réactif de Jones pour accéder aux acides.
i)
j) Les réactions de transformation de radical formyle en radical carbamoyle et de radical carbamoyle en radical nitrile, sont réalisées notamment pour R₃ et R₄ selon les conditions usuelles connues de l'homme du métier, telles que par exemple passage par le céto nitrile et déplacement par une amine (Chem. Comm. 1971, p. 733).
k) Les éventuelles fonctions nitrile des produits décrits ci-dessus peuvent être, si désiré, transformées en tétrazolyle dans les conditions usuelles connues de l'homme du métier telles que par exemple par cycloaddition d'un azidure métallique tel que par exemple l'azidure de sodium ou un azidure de trialkylétain sur la fonction nitrile ainsi qu'il est indiqué dans la méthode décrite dans l'article référencé comme suit :J. Organometallic Chemistry., 33, 337 (1971) KOZIMA S.& coll.
   On peut noter que la réaction de transformation d'un carbamate en urée et notamment d'un sulfonylcarbamate en sulfonylurée, peut être réalisée par exemple au reflux d'un solvant comme par exemple le toluène en présence de l'amine adéquate.
l) la transformation d'un radical halogéné en radical formyle peut être notamment effectuée par action d'un dérivé organo métallique, par exemple le bromure d'éthyl magnésium, au sein d'un solvant organique,
m) la transformation du radical formyle en radical CH=CH-CO₂alk peut être effectuée par une réaction de type Wittig par condensation d'un sel de phosphonium approprié en présence d'hydrure de sodium ; la transformation en acide est effectuée par hydrolyse, par exemple au moyen d'une base telle que la soude en milieu alcoolique
n) la transformation du radical formyle en radical

   -CH=CH-A'

   peut être effectuée par réaction de Wittig comme indiqué ci-dessus ; la transformation en radical

   -CH₂-CH₂-A'

   est effectuée par réduction, au moyen d'hydrogène en présence d'un catalyseur, par exemple l'oxyde de platine,
   On peut noter que la transformation du radical formyle en radical CH₂OH peut être réalisée au moyen d'un agent réducteur, par exemple le borohydrure de sodium dans l'éthanol à la température ambiante ; la transformation en radical -CH₂-SR peut être effectuée par action du thiol approprié R-SH sur le mésylate intermédiaire préparé au préalable par action du chlorure de mésyle sur l'alcool en présence de la base de Hunig,
o) l'oxydation du substituant S-alk en sulfoxyde peut être effectuée par exemple, par action de l'acide métachloroperbenzoique ; la transformation du thiol est obtenue par la réaction de PUMMERER par exemple en présence d'anhydride trifluoroacétique ; la transformation du substituant SH en SZ₂ peut être obtenue par action d'un dérivé halogène Hal-Z₂ par exemple l'iodocyclohexane.
   Il est entendu que les réactions décrites ci-dessus peuvent être effectuées selon les méthodes usuelles connues de l'homme du métier.
p) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.
   Le groupement phtalimido peut être éliminé par l'hydrazine.
   On trouvera une liste de différents groupemente protecteurs utilisables par exemple dans le brevet BF 2 499 995.
q) Les produits décrits ci-dessus peuvent, si désiré, faire l'objet de réactions de salification par exemple par un acide minéral ou organique ou par une base minérale ou organique selon les méthodes usuelles connues de l'homme du métier.
r) Les éventuelles formes optiquement actives des produits décrits ci-dessus peuvent être préparées par dédoublement des racémiques selon les méthodes usuelles connues de l'homme du métier.

Des illustrations de telles réactions définies ci-dessus sont données dans la préparation des exemples décrits ci-après.

Les composés de l'invention tels que définis ci-dessus ainsi que leurs sels d'addition avec les acides présentent d'intéressantes propriétés pharmacologiques.

Les produits de l'invention tels que définis ci-dessus, sont doués de propriétés antagonistes pour les récepteurs à l'endothéline et sont ainsi notamment inhibiteurs des effets de l'endothéline, notamment des effets vasoconstricteurs et hypertenseurs induits par l'endothéline. On note en particulier un effet antiischémique, l'activité vasoconstrictrice de l'endothéline étant abolie.

Les produits de l'invention sont également capables de s'opposer aux effets stimulants de l'endothéline au niveau de tous les types cellulaires, notamment les cellules musculaires lisses, les fibroblastes, les cellules neuronales et les cellules osseuses.

Ces propriétés justifient leur application en thérapeutique et l'invention a particulièrement pour objet à titre de médicaments, les produits de l'invention,
lesdits produits de l'invention étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de l'invention

L'invention décrit ci-dessus un procédé de préparation des produits de l'invention dans laquelle R₁ représente un radical méthoxyméthyle, éthyle, n-propyle, isopropyle, n-butyle, isobutyle, terbutyle, cyclopentyle, cyclohexyle, dioxolane, dioxane, dithiolane, thiényle ou furyle, R₂ représente soit un radical alkylthio renfermant de 3 à 10 atomes de carbone substitué par un radical carboxy libre, salifié ou estérifié ou par un radical tétrazolyle libre ou salifié, soit un radical cyclohexylthio, cyclopentylthio ou pipéridinylthio substitué par un radical carboxy libre, salifié ou estérifié ; par un radical tétrazolyle libre ou salifié ; ou par un radical alkyle, alkényle, alcoxy ou alkylthio renfermant au plus 4 atomes de carbone eux-mêmes substitués par un radical carboxy libre, salifié ou estérifié ou par un radical tétrazolyle libre ou salifié, R₃ représente un radical carboxy libre, salifié ou estérifié ou un radical tétrazolyle libre ou salifié, et Y représente un radical phényle substitué par un radical dioxol et éventuellement substitué par un atome d'halogène, lesdits produits de l'invention étant sous toutes les formes isoméres possibles racémiques ou optiquement actives, ainsi que les sels d'addition avec les acides minéraux et organiques ou avec les bases minérales et organiques pharmaceutiquement acceptables desdits produits de l'invention.

L'invention a pour objet, à titre de médicaments, les produits décrits ci-après dans les exemples répondant aux formules suivantes :
- acide 4-((4-(carboxymèthylène) cyclohexyl) thio))-1-((6-chloro-1,3-benzodioxol-5-yl)méthyl)-2-propyl-1H-imidazole-5-carboxylique,
- acide 4-((5-carboxypentyl) thio)-1-((6-chlcro-1,3-benzodioxol-5-yl) méthyl)-2-(1,3-dioxolan-2-yl)-1H-imidazole-5-carboxylique,
- acide 4-((4-(carboxyméthyl) cyclohexyl) thio)-1-((6-chloro-1,3-benzodioxol-5-yl) méthyl)-2-propyl-1H-imidazole-5-carboxylique,
- acide 1-((6-chloro 1,3-benzodioxol-5-yl) méthyl) 4-((4-carboxycyclohexyl) thio) 2-propyl 1H-imidazole 5-carboxylique,
- acide 4(((4-carboocycyclohexyl) méthyl) thio) 1-((6-chloro 1,3-benzodioxol-5-yl) méthyl) 2-propyl 1H-imidazole 5-carboxylique,
- acide trans 4-((4-(carboxyméthyl) cyclohexyl) thio) 1-((6-chloro 1,3-benzodioxol-5-yl) méthyl) 2-(1,3-dioxolan-2-yl) 19-imidazole 5-carboxylique,
- acide 4-((4-(carboxyméthyl) cyclohexyl) thio) 1-((6-chloro 1,3-benzodioxol-5-yl) méthyl) 2-(2-furanyl) 1H-imidazole 5-carboxylique,
- acide trans 4-((4-(carboxyméthyl) cyclohexyl) thio) 1-((6-chloro 1,3-benzodioxol-5-yl) méthyl) 2-cyclopentyl 1H-imidazole 5-carboxylique,
- acide 4-((4-carboxycyclohexyl) thio) 1-((6-chloro 1,3-benzodioxol-5-yl) méthyl) 2-(2-furanyl) 1H-imidazole 5-carboxylique,
- sel de disodium de l'acide 4-((4-(carboxyméthyl) cyclohexyl) thio) 1-((6-chloro 1,3-benzodioxol-5-yl) méthyl) 2-propyl 1H-imidazole 5-carboxylique,
- sel de dipotassium de l'acide 4-((4-(carboxyméthyl) cyclohexyl) thio) 1-((6-chloro 1,3-benzodioxol-5-yl) méthyl) 2-propyl 1H-imidazole 5-carboxylique.
ainsi que leurs sels d'addition avec les acides minéraux ou organiques ou avec les bases minérales et organiques pharmaceutiquement acceptables.

Les médicaments, objet de l'invention, trouvent, par exemple, leur emploi dans le traitement de tous les spasmes vasculaires, dans le traitement du vasospasme consécutif à une hémorragie cérébrale, dans le traitement des spasmes coronariens, des spasmes vasculaires périphériques ainsi que dans le traitement des insuffisances rénales. Ces médicaments peuvent également être utilisés dans le traitement de l'infarctus du myocarde, de l'insuffisance cardiaque congestive, dans la prévention des resténoses post-angioplastie, des fibroses cardiaques et vasculaires, dans le traitement de l'athérosclérose, de certaines formes d'hypertension comme notamment l'hypertension pulmonaire, ainsi que dans le traitement de l'asthme.

Les médicaments, objet de l'invention, peuvent également trouver une application dans le traitement de l'ostéoporose, de l'hyperplasie prostatique et en tant que protecteurs neuronaux.

L'invention s'étend aux compositions pharmaceutiques contenant à titre de principe actif l'un au moins des médicaments tels que définis ci-dessus.

Ces compositions pharmaceutiques peuvent être administrées par voie buccale, rectale, par voie parentérale ou par voie locale en application topique sur la peau et les muqueuses ou par injection par voie intraveineuse ou intramusculaire.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 1 à 300 mg par jour chez l'adulte, par voie orale ou de 1 à 100 mg par jour par voie intraveineuse.

Certains produits de départ de formules (II) et (XVI) sont connus et peuvent être préparés par exemple comme indiqué dans le brevet européen EP 168 950.

D'autres produits de départ de formules (II) et (XVI) peuvent en particulier être préparés comme indiqué dans le brevet européen EP 0465368, ou encore dans les exemples décrits ci-après dans la partie expérimentale.

Certains produits de départ de formules (II) et (XVI) sont commerciaux tels que par exemple, les produits de formule (II) suivants :
- le 2-propylimidazole, le 2-isopropylimidazole, le 2-éthylimidazole, le 2-méthylimidazole.

Des exemples de produits commerciaux de formule (XVI) sont donnés dans les brevets EP 0465368 ou EP 0503162.

On peut encore notamment préparer certains produits de formules (II) et (XVI) à partir de produits de formule (II) par exemple en les soumettant à une ou plusieurs des réactions décrites ci-dessus en a) à r), réalisées dans les conditions également décrites ci-dessus.

Certains produits de formules (XVI) peuvent également être obtenus par monohalogénation du produit de formule (II) tel que défini ci-dessus en produit de formule (P₁) : 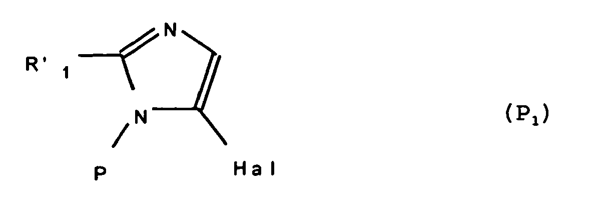 dans lequel R'₁ et P ont les significations indiquées ci-dessus pour le produit de formule (II), produit de formule (P₁) que l'on peut faire réagir, après échange suivant la réaction halogène métal connue de l'homme du métier, avec l'électrophile adapté, suivant les méthodes connues de l'homme du métier et notamment par exemple suivant le même type de réaction décrite ci-dessus pour passer par exemple du composé de formule (IX) au composé de formule (XI).
- Certains produits de formule (XVI) peuvent aussi être préparés selon le schéma suivant : 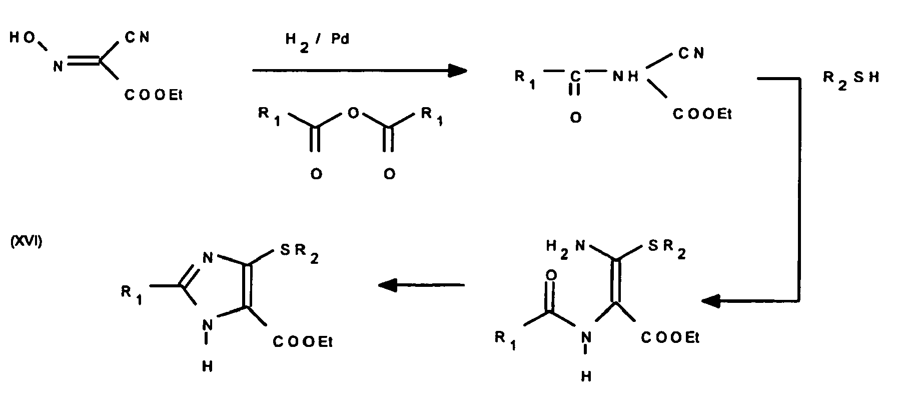
- Certains produits de formule (XVI) peuvent également être préparés selon le schéma suivant : 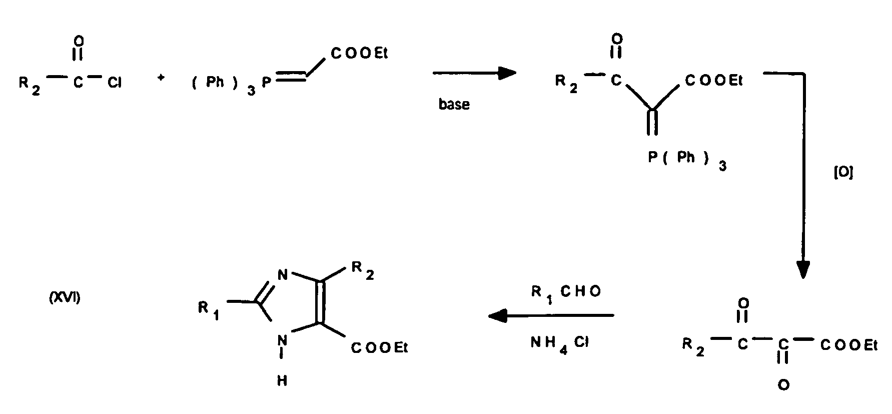
- Certains produits de formule (XVI) peuvent encore être préparés selon le schéma suivant :

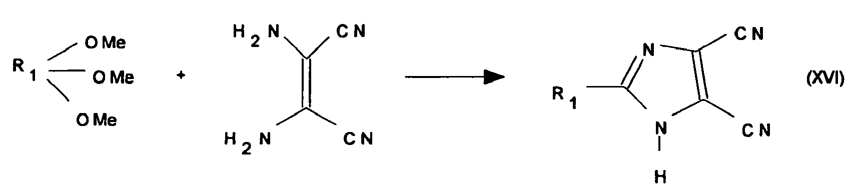

Les composés de départ de formule (VI) tel que le 2,4,5-tribromoimidazole ou encore les produits de départ de formules (XX) et (XXI) peuvent être disponibles dans le commerce ou peuvent être préparés selon les méthodes usuelles connues de l'homme du métier.

Les produits de départ de formule (X), sont commerciaux tels que notamment :
- le méthyl chloroformate
- le benzyl chloroformate
- l'isobutyl chloroformate
- l'éthyl chloroformate
- le N-propyl chloroformate

Les produits de départ de formules (VIIIa) et (VIIIb) sont commerciaux tels que notamment :
les produits de formule (VIIIa) suivants :
   - le benzaldehyde ou butanal
les produits de formule (VIIIb) suivants :
   - le chlorure de benzoyle ou de buturyle.

Un procédé de préparation de certains produits de formule (III) est notamment décrit dans le brevet européen EP 0465368.

Des exemples de préparation de composés de formule (III) sont également décrits dans la littérature et des exemples en sont donnés notamment dans le brevet US 4,880,804 ou par exemple dans la référence Chemistry and Industry 7 september 19 8 7 HOWARD and COLQUHOUN pp. 612-617.

Notamment, le produit de formule (III) qui est le chlorure de 6-chloro pipéronyle est commercial chez ACROS.

L'invention a enfin pour objet à titre de produits industriels nouveaux, les composés de formules (IV), (V), (IX), (XI) et (XIII) dans lesquels, étant entendu que dans les composés de formules (IX) et (XI), W représente le radical CH₂-Y', Y' représente le radical phényle substitué par un radical dioxol et éventuellement substitué par un autre substituant choisi parmi les atomes d'halogène et les radicaux alcoxy et alkyle renfermant au plus 4 atomes de carbone et dans lesquelles les éventuelles fonctions réactives sont éventuellement protégées par des groupements protecteurs.

L'invention a ainsi particulièrement pour objet l'utilisation des produits de l'invention telle que définie ci-dessus, pour la préparation de compositions pharmaceutiques destinées au traitement d'affections résultant d'une stimulation anormale des récepteurs de l'endothéline et notamment destinées au traitement de l'hypertension induite par l'endothéline, de tous les spasmes vasculaires, au traitement des suites d'une hémorragie cérébrale, des insuffisances rénales, de l'infarctus du myocarde, de l'insuffisance cardiaque et à la prévention des resténoses post-angioplastie ainsi que des fibroses cardiaques et vasculaires.

Les exemples suivants illustrent l'invention sans toutefois la limiter.

### PREPARATION 1 : 4-bromo-cyclohexaneacétate d'éthyle

### STADE 1 : 2-(4-bromocyclohexylidène)-acétate d'éthyle

On introduit 8,07 g de triéthylphosphonoacétate dans 20 ml de tétrahydrofuranne, refroidit à 0°C et ajoute 40 ml d'une solution molaire de lithium hexaméthyldisilazane dans le tétrahydrofuranne. Après 15 minutes on ajoute 6,5 g de 4-bromo-cyclohexanone (préparé ainsi qu'il est décrit dans Heterocycles, vol. 18, 1982 p. 163-167) et laisse 1 heure à température ambiante. On hydrolyse avec une solution saturée de chlorure d'ammonium, extrait à l'acétate d'éthyle et obtient après chromatographie 8,5 g du produit attendu (huile incolore).

### STADE 2 : 4-bromo-cyclohexaneacétate d'éthyle

On introduit 3 g de produit obtenu au stade 1 ci-dessus, dans 100 ml d'éthanol, hydrogène en présence d'oxyde de platine comme catalyseur pour donner après filtration 2,9 g de produit attendu (huile incolore). Les exemples indiqués ci-après de référence représentent des exemples de mode de réalisation de l'invention.

### EXMPLE 1 de référence: 1-[(6-chloro-1,3-benzodioxol-5-yl)-méthyl]-4-[[4-(2-éthoxy-2-oxoéthyl)cyclohexyl]thio]-2-propyl-1H-imidazole-5-carboxylate d'éthyle

### STADE 1 : cyano-[(1-oxobutyl) amino] acétate d'éthyle

On mélange 5 g d'éthyl (hydroxyimino) cyanoacétate, 40 cm³ de tétrahydrofuranne, 11,5 cm³ d'anhydride butyrique et 2,5 g de platine et agite en atmosphère d'hydrogène jusqu'à saturation. On filtre, rince par 5 x 15 cm³ d'éther éthylique, évapore l'éther, ajoute peu à peu 200 cm³ d'essence G, essore, lave avec 3 x 10 cm³ d'essence G et sèche à environ 75°C. On concentre à - 10 cm³, ajoute 50 cm³ d'essence G, laisse cristalliser 30 mn à température ambiante, essore, lave avec 3 x 3 cm³ d'essence G et sèche à environ 75°C. On obtient 5,73 g de produit. PF = 110°C.

### Recristallisation pour analyses :

On dissout 540 mg du produit obtenu dans 50 cm³ d'éther isopropylique à reflux, filtre, concentre, laisse environ 1 h au repos à température ambiante, essore, lave à l'éther isopropylique et sèche. On obtient 440 mg de produit attendu. PF = 110°C.

### Microanalyse pour C₉H₁₄N₂O₃ = 198,22

| | C | H | N O | |
|---|---|---|---|---|
| % calculé | 54,53 | 7,12 | 14,13 | 24,22 |
| % trouvé | 54,5 | 7,2 | 14,0 | - |

### Spectre IR CHCl₃

| | |
|---|---|
| =C-NH | ~ 3430 cm⁻¹ |
| -C≡N | ~ 2245 cm⁻¹ |
| C=O | 1758 cm⁻¹ ester |
| | 1692 cm⁻¹ amide |
| Amide II | 1492 cm⁻¹ |

### Stade 2 : 3-amino 2-[(1-oxobutyl) amino] 3-(méthylthio) 2-propenoate d'éthyle

A une solution de 20 g de nitrile obtenu au stade A ci-dessus, dans 400 ml d'éthanol, on ajoute 1,4 ml de triéthylamine, on refroidit à environ -10°C et introduit environ 22 g de méthylmercaptan par barbotage. On agite environ 72 heures à 0°C. On élimine l'excès de méthanethiol chasse l'éthanol, empâte à l'essence G, filtre et sèche. On obtient 24,3 g de produit attendu (cristaux incolores).
F_{K115} = 120-124°C

### Microanalyse pour C₁₀H₁₈N₂O₃S = 246,33

| | C | H | N | S | O |
|---|---|---|---|---|---|
| % calculé | 48,76 | 7,37 | 11,37 | 13,02 | 19,49 |
| % trouvé | 48,6 | 7,5 | 11,4 | 12,6 | - |

### Spectre IR CHCl₃

| | |
|---|---|
| =C-NH₂ | 3500, 3412 cm⁻¹ |
| =C-NH | 3365, 3275 cm⁻¹ |
| C=O complexe | 1665 cm⁻¹ |
| C=C et NH₂ déf. | 1592 cm⁻¹ |
| Amide II | 1488 cm⁻¹ |

### Spectre UV dans EtOH

| | |
|---|---|
| Max. 220 nm | ∈ = 5500 |
| Max. 291-292 | ∈ = 19400 |

### Stade 3 : 4-(méthylthio) 2-propyl 1H-imidazole 5-carboxylate d'éthyle

A 20,1 g de pentachlorure de phosphore dans 300 cm³ de chlorure de méthylène, refroidis à environ -70°C on ajoute une solution de 12,9 g de 4-diméthylaminopyridine dans 90 cm³ de chlorure de méthylène.

On maintient encore environ 15 mn à environ -70°C puis on introduit une solution de 12 g du produit obtenu au stade B ci-dessus dans 120 cm³ de chlorure de méthylène. On laisse revenir à température ambiante et maintient sous agitation pendant environ 22 heures.

On verse le mélange réactionnel dans 2,5 1 d'eau + glace et neutralise par addition d'environ 60 g de bicarbonate de sodium. On agite encore environ 30 mn, on décante et on extrait avec 500 cm³ de chlorure de méthylène. On lave à l'eau salée, sèche et chasse le solvant à environ 50°C. On purifie par chromatographie sur silice avec pour éluants chlorure de méthylène-acétate d'éthyle (90-10) puis chlorure de méthylène-acétate d'éthyle (80-20). On chasse les solvants à environ 50°C, empâte à l'essence G, filtre et sèche. On obtient 7,4 g de produit attendu (cristaux incolores). F_{K95} = 85°C.

### Microanalyse

| Concordance pour C₁₀H₁₆N₂O₂S = 228,32 | | | | | |
|---|---|---|---|---|---|
| | C | H | N | S | O |
| % calculé | 52,61 | 7,06 | 12,27 | 14,04 | 14,02 |
| % trouvé | 52,7 | 7,3 | 12,2 | 14,0 | |

### Spectre IR CHCl₃

| | |
|---|---|
| =C-NH | 3440 - 3260 cm⁻¹ |
| C=O Complexe max. | ~ 1672 cm⁻¹ |
| Hétérocycle | 1542 - 1498 cm⁻¹ |

### Spectre UV dans EtOH

| | |
|---|---|
| Max. 213-214 nm | ∈ = 14500 |
| Infl. 229 nm | ∈ = 7200 |
| Max. 286 nm | ∈ = 12200 |

### Spectre UV dans EtOH/HCl N/10

| | |
|---|---|
| Max. 238 nm | ∈ = 6800 |
| Max. 277 nm | ∈ = 9600 |
| par retour basique --> | max. 296 nm. |

### STADE 4 : 1-[(6-chloro-1,3-benzodioxol-5-yl)méthyl]-4-(méthylthio)-2-propyl-1H-imidazole-5-carboxylate d'éthyle

On introduit 10 g du produit obtenu au stade 3 ci-dessus et 13,5 g de chlorure de 6-chloro pipéronyl dans 100 ml de diméthylformamide, ajoute 9 g de carbonate de potassium et chauffe à 100°C pendant une heure. On refroidit à température ambiante, puis verse le milieu réactionnel sur de la glace, filtre le précipité obtenu puis l'empâte avec de l'éther isopropylique. On obtient ainsi 9,25 g de produit attendu. STADE 5 : 1-[(6-chloro-1,3-benzodioxol-5-yl)méthyl]-4-(méthylsulfinyl)-2-propyl-1H-imidazole-5-carboxylate d'éthyle

On introduit 9,26 g du produit obtenu au stade 4 ci-dessus dans 400 ml de chlorure de méthylène à 0°C et ajoute 5,8 g d'acide métachloroperbenzoïque. On agite une heure à température ambiante et lave le milieu réactionnel avec de l'hydrure de sodium et de potassium, extrait au chlorure de méthylène, lave à l'eau, sèche, évapore le solvant. On obtient après chromatographie 8,8 g de produit attendu. F = 187°C.

### STADE 6 : 1-[(6-chloro-1,3-benzodioxol-5-yl)méthyl]-4-mercapto-2-propyl-1H-imidazole-5-carboxylate d'éthyle

On introduit 800 mg du produit obtenu au stade 5 ci-dessus dans 16 ml de chlorure de méthylène et on ajoute 0,54 ml d'anhydride trifluoroacétique. On agite pendant 30 minutes, évapore à sec et reprend le résidu dans 10 ml de méthanol et 4 ml de triéthylamine. On agite 30 minutes, extrait au chloroforme, lave avec une solution saturée de chlorure d'ammonium, sèche et évapore à sec. On obtient ainsi 720 mg de produit attendu.

### STADE 7 : 1-[(6-chloro-1,3-benzodioxol-5-yl)méthyl]-4-[[4-(2-éthoxy-2-oxoéthyl)cyclohexyl]thio]-2-propyl-1H-imidazole-5-carboxylate d'éthyle

On introduit 1 g du produit obtenu au stade 6 ci-dessus dans du diméthylformamide, ajoute 146 mg d'une suspension d'hydrure de sodium à 60 % dans l'huile et laisse agiter 15 minutes à température ambiante. On ajoute ensuite 1,08 mg du produit obtenu au stade 2 de la préparation 1, et laisse agiter 24 h à température ambiante. On hydrolyse avec une solution saturée de chlorure d'ammonium et extrait à l'acétate d'éthyle. On obtient ainsi après chromatographie 1,1 g de produit attendu (solide amorphe).

### EXEMPLE 2 : Acide 4-((4-(carboxyméthyl) cyclohexyl) thio)-1-((6-chloro-1,3-benzodioxol-5-yl) méthyl)-2-propyl-1H-imidazole-5-carboxylique

On introduit 760 mg du produit de l'exemple 1, dans 20 ml de tétrahydrofuranne, ajoute 50 ml d'éthanol et 25 ml de soude 2N et laisse 24 h à température ambiante. On évapore le tétrahydrofuranne et l'éthanol puis acidifie le milieu réactionnel à pH 1. On extrait à l'acétate d'éthyle, évapore à sec et recristallise le résidu dans l'éthanol. On obtient 490 mg de produit attendu. F = 166°C.

### EXEMPLE 3 : Acide 4-((5-(carboxypentyl) thio)-1-((6-chloro 1,3-benzodioxol-5-yl) méthyl)-2-(1,3-dioxolan-2-yl)-1H-imidazole-5-carboxylique

### STADE 1 : 1-((6-chloro-1,3-benzodioxol-5-yl)-méthyl)-2,4,5-tribromo-1H-imidazole

On introduit 25 g de 2,4,5-tribromoimidazole dans 500 ml de diméthylformamide et ajoute 4,3 g d'hydrure de sodium. L'agitation est maintenue 10 mn à température ambiante. On ajoute ensuite au milieu réactionnel 18,4 g de chlorure de 6-chloro pipéronyl, puis 25 g d'iodure de sodium et l'agitation est poursuivie pendant 15 mn à température ambiante.

Le milieu réactionnel est finalement versé sur 3 l d'eau, on essore, lave abondamment par de l'eau, puis successivement par 250 ml d'éthanol, 250 ml d'isopropanol, puis finalement par 250 ml d'éther isopropylique.

On sèche et récupère 31,5 g de produit attendu (solide
crème) Pf = 225°C.
IR CHC13 (cm⁻¹)
Absence de =C-NH
Hétérocycle aromatique 1624-1506-1497-1485

### STADE 2 : 4,5-dibromo-1-[(6-chloro-1,3-benzodioxol-5-yl)-méthyl]-1H-imidazole-2-carboxaldéhyde

On introduit 20 g du produit obtenu au stade 1 ci-dessus dans 200 ml de chlorure de méthylène anhydre auquel on ajoute 500 ml d'éther éthylique anhydre. On ajoute une solution 3M dans l'éther éthylique de bromure d'éthyl magnésium et laisse agiter 20 mn à température ambiante. On ajoute alors un excès (15 ml) de diméthylformamide anhydre. Après 2 heures à température ambiante on hydrolyse avec 200 ml d'acide chlorhydrique 1N et extrait 3 fois avec 300 ml d'acétate d'éthyle. La phase organique est lavée avec du chlorure de sodium saturé puis séchée et évaporée à sec. On obtient ainsi 14,5 g de produit attendu.

### STADE 3 : 4,5-dibromo-1-[(6-chloro-1,3-benzodioxol-5-yl) méthyl]-2-(1,3-dioxolan-2yl)-1H-imidazole

On introduit le produit obtenu au stade 2 ci-dessus dans 200 ml de toluène, ajoute 20 ml d'éthylène glycol et porte à reflux pendant une nuit puis évapore à sec. On ajoute 200 ml d'une solution saturée d'hydrogénocarbonate de sodium que l'on extrait 3 fois par 200 ml d'acétate d'éthyle. La phase organique est lavée avec du chlorure de sodium saturé puis séchée et évaporée à sec. Le résidu obtenu est empâté dans l'éther isopropylique, filtré et séché. On obtient ainsi 13,5 g de produit attendu (solide). Pf = 188°C.

### STADE 4 : 4-bromo-1-[(6-chloro-1,3-benzodioxol-5-yl)méthyl]-2-(1,3-dioxolan-2-yl)-1H-imidazole-5-carboxaldéhyde

On introduit 10 g de produit obtenu au stade 3 ci-dessus, dans 200 ml de tétrahydrofuranne anhydre. On ajoute 10 ml de bromure d'éthyle magnésium (solution 3M dans l'éther éthylique) et on laisse agiter 20 mn à température ambiante. On ajoute alors un excès de diméthylformamide anhydre. Après 2 heures à température ambiante, on hydrolyse avec 200 ml d'acide chlorhydrique 1N et on extrait 3 fois avec 300 ml d'acétate d'éthyle. La phase organique est lavée avec du chlorure de sodium saturé puis séchée et évaporée à sec. Le résidu obtenu est empâté dans l'éther isopropylique. On obtient ainsi 7,6 g de produit attendu, utilisé sans purification pour l'étape suivante.

### STADE 5 : 6-[[1-[(6-chloro-1,3-benzodioxol-5-yl)méthyl]-2-(1,3-dioxolan-2-yl)-5-formyl-1H-imidazol-4-yl]thio]-hexanoate d'éthyle

On introduit 3,9 g de 6-mercaptohexanoate d'éthyle dissous dans 200 ml de diméthylformamide anhydre, on ajoute 1,1 g d'hydrure de sodium à 50 % dans l'huile et laisse 20 mn à température ambiante. On ajoute ensuite 7,6 g du produit obtenu au stade 4 ci-dessus, et laisse une nuit à température ambiante. On évapore le diméthylformamide sous vide, reprend le résidu dans 200 ml de chlorure d'ammonium saturé et extrait avec trois fois 200 ml de chlorure de méthylène. On sèche les phases organiques, filtre et évapore à sec. Le résidu est empâté à l'éther isopropylique, filtré. On obtient ainsi 5,8 g de produit attendu (solide).

### STADE 6 : Acide 4-((5-(carboxypentyl) thio) 1-((6-chloro 1,3-benzodioxol-5-yl) méthyl) 2-(1,3-dioxolan-2-yl)-1H-imidazole-5-carboxylique

On introduit 5,1 g du produit obtenu au stade 5 ci-dessus dans 100 ml de chlorure de méthylène, ajoute 500 ml de méthanol puis par fractions : 22 g d'oxyde de manganèse, 2,2 g de cyanure de sodium et 1,5 ml d'acide acétique. On laisse agiter 72 heures à température ambiante, puis on filtre, lave au chlorure de méthylène. La phase organique est lavée avec du chlorure de sodium saturé puis séchée et évaporée à sec. Le résidu obtenu est chromatographié sur silice pour donner 3,1 g d'ester méthylique intermédiaire qui est saponifié dans un mélange de soude 2N/éthanol pour donner 2,5 g de produit attendu. F = 175°C.

### EXEMPLE 4 de référence: Acide 4-((5-(carboxypentyl) thio) 1-((6-chloro 1,3-benzodioxol-5-yl) méthyl) 2-(méthoxyméthyl)-1H-imidazole-5-carboxylique

### STADE 1 : 4,5-dibromo-1-[(6-chloro-1,3-benzodioxol-5-yl) méthyl]-2-(méthoxyméthyl)-1H-imidazole

On introduit 28,2 g du produit obtenu au stade 1 de l'exemple 3 dans 400 ml d'éthanol, ajoute 2,5 g de borohydrure de sodium et après 2 heures, ajoute 10 ml d'acide acétique. On agite 15 minutes, évapore à sec, empâte le résidu avec de l'éther isopropylique, filtre et obtient 27,5 g d'un solide crème. On dissout les 27,5 g obtenus précédemment dans 400 ml de tétrahydrofuranne, ajoute 3,73 g d'hydrure de sodium à 50 % dans l'huile, agite 15 mn et ajoute 4,5 ml d'iodure de méthyle. On laisse 1 nuit à température ambiante, extrait à l'acétate d'éthyle et après chromatographie, obtient 18,7 g de produit attendu.

### STADE 2 : Acide 4-((5-(carboxypentyl) thio)-1-((6-chloro 1,3-benzodioxol-5-yl) méthyl) 2-(méthoxyméthyl)-1H-imidazole-5-carboxylique

On procède comme aux stades 4, 5 et 6 de l'exemple 3 à partir du produit obtenu au stade 1 ci-dessus et obtient ainsi le produit attendu.
F = 170°C.

### EXEMPLE 5 de référence: 1-[(6-chloro-1,3-benzodioxol-5-yl)-méthyl]-4-[(6-éthoxy-6-oxohexyl)thio]-2-propyl-1H-imidazole-5-carboxylate d'éthyle

On procède comme au stade 7 de l'exemple 1 à partir du produit obtenu au stade 6 de l'exemple 1 et du 6-bromo-hexanoate d'éthyle au lieu du 4-bromo-cyclohexaneacétate d'éthyle. On obtient ainsi le produit attendu.

### EXEMPLE 6 de référence: Acide 4-((5-(carboxypentyl) thio) 1-((6-chloro 1,3-benzodioxol-5-yl) méthyl) 2-propyl-1H-imidazole-5-carboxylique

On procède comme à l'exemple 2 à partir du produit de l'exemple 5 et obtient ainsi le produit attendu.
F = 145°C.

### EXEMPLE 7 de référence: 1-[(6-chloro-1,3-benzodiaxol-5-yl)-méthyl]-4-[(5-éthoxy-5-oxopentyl)thio]-2-propyl-1H-imidazole-5-carboxylate d'éthyle

On procède comme au stade 7 de l'exemple 1 à partir du produit obtenu au stade 6 de l'exemple 1 et du 5-bromo-pentanoate d'éthyle au lieu du 4-bromo-cyclohexaneacétate d'éthyle. On obtient ainsi le produit attendu.

### EXEMPLE 8 de référence: Acide 4-((4-carboxybutyl) thio)-1-((6-chloro-1,3-benzodioxol-5-yl) méthyl)-2-(propyl)-1H-imidazole-5-carboxylique

On procède comme à l'exemple 2 à partir du produit de l'exemple 7 et obtient ainsi le produit attendu.
F = 156°C.

### EXEMPLE 9 de référence: 1-[(6-chloro-1,3-benzodioxol-5-yl)-méthyl]-4-[[4-(2-éthoxy-2-oxoéthylidène)cyclohexyl]thiol]-2-propyl-1H-imidazole-5-carboxylate d'éthyle

On introduit 1,1 g du produit obtenu au stade 6 de l'exemple 1, ajoute 127 mg d'une suspension d'hydrure de sodium à 50 % dans l'huile et laisse agiter 15 minutes à température ambiante. On ajoute ensuite 657 mg du produit obtenu au stade 1 de la préparation 1 et laisse agiter 24 heures à température ambiante. On hydrolyse avec une solution saturée de chlorure d'ammonium et on extrait à l'acétate d'éthyle et obtient après chromatographie 880 mg du produit attendu (solide amorphe).

### EXEMPLE 10 : Acide 4-((4-(carboxyméthylène) cyclohexyl) thio)-1-((6-chloro 1,3-benzodioxol-5-yl) méthyl)-2-propyl-1H-imidazole-5-carboxylique

On introduit 880 mg du produit de l'exemple 9 dans 20 ml de tétrahydrofuranne, ajoute 50 ml d'éthanol et 25 ml de soude 2N et laisse 24 heures à température ambiante. On évapore le tétrahydrofuranne et l'éthanol puis on acidifie le milieu réactionnel à pH 1. On extrait à l'acétate d'éthyle, évapore à sec et recristallise le résidu dans l'acétate d'éthyle. On obtient ainsi 490 mg de produit attendu.
F = 168°C.

### EXEMPLE 11 de référence: 2-butyl-1-[(6-chloro-1,3-benzodioxol-5-yl)méthyl]-4[(6-ethoxy-6-oxohexyl)thio]-1H-imidazole-5-carboxylate d'éthyle

On procède comme à l'exemple 1, en utilisant au stade 4 de l'exemple 1 à la place du 2-n-propyl-4-(méthylthio)-1H-imidazole-5-carboxylate d'éthyle, le 2-n-butyl-4-(méthylthio)-1H-imidazole-5-carboxylate d'éthyle, obtenu ainsi qu'il est indiqué au stade d) de l'exemple 56 de EP 0503162 et en utilisant au stade 7 de l'exemple 1, à la place du 4-bromo-cyclohexaneacétate d'éthyle, du 6-bromohexanoate d'éthyle. On obtient ainsi le produit attendu, sous forme d'une huile jaune.

### EXEMPLE 12 de référence: Acide 2-butyl 4-((5-carboxypentyl) thio) 1-((6-chloro 1,3-benzodiaxol-5-yl) méthyl)-1H-imidazole-5-carboxylique

On procède comme à l'exemple 2 à partir du produit de l'exemple 11. On obtient ainsi le produit attendu.
F = 128°C.

En opérant comme au stade 7 de l'exemple 1 et à l'exemple 2, en utilisant au départ le 1-((6-chloro 1,3-benzodioxol-5-yl) méthyl) 4-mercapto 2-propyl 1H-imidazole 5-carboxylate d'éthyle obtenu comme indiqué à l'exemple 1 stade 6 et le dérivé halogéné ou tosylé approprié, on a préparé les produits des exemples suivants :

### EXEMPLE 13 : Acide 1-((6-chloro 1,3-benzodioxol-5-yl) méthyl) 4-((4-carboxycyclohexyl) thio) 2-propyl 1H-imidazole 5-carboxylique (cis/trans).

**F = 174°C.**

### EXEMPLE 14 : Acide 4-(((4-carboxycyclohexyl) méthyl) thio) 1-((6-chloro 1,3-benzodioxol-5-yl) méthyl) 2-propyl 1H-imidazole 5-carboxylique (cis/trans).

F = 184°C.

### EXEMPLE 15 de réference: Acide 4-(((3-carboxycyclohexyl) méthyl) thio) 1-((6-chloro 1,3-benzodioxol-5-yl) méthyl) 2-propyl 1H-imidazole 5-carboxylique (diastéréoisomères).

F = 178°C.

### EXEMPLE 16 de réference: Acide cis 4-(((2-((carboxyméthoxy) méthyl) cyclohexyl) méthyl) thio) 1-((6-chloro 1,3-benzodioxol-5-yl) méthyl) 2-propyl 1H-imidazole 5-carboxylique (diastéréoisomères).

F = 172°C.

### Prépararation des réactifs utilisés au départ des exemples 13 à 16.

### 1) Préparation du 4-bromo cyclohexyl carboxylate d'éthyle utilisé au départ de l'exemple 13.

On introduit mentement 0,9 ml de tribromure de phosphore dans 5 ml de (4-hydroxycyclohexyl) carboxylate d'éthyle et chauffe le milieu réactionnel à 80°C pendant 6 heures. On laisse revenir à température ambiante, lave à l'eau salée, extrait à l'acétate d'éthyle, sèche et évapore le solvant sous pression réduite. Après chromatographie du résidu sur silice (éluant : AcOEt-cyclohexane 10-90), on obtient 3,51 g de produit attendu.

### 2) Préparation du 1-((4-tosyloxyméthyl) cyclohexyl) carboxylate d'éthyle utilisé au départ de l'exemple 14.

On agite 5 heures à température ambiante 1 g de 1,4-dihydroxyméthyl cyclohexane dans 10 ml de diméthylformamide en présence de 0,94 g d'imidazole et 1,8 ml de chlorure de diphényl triméthyl silane puis verse dans une solution aqueuse saturée en chlorure d'ammonium, extrait à l'acétate d'éthyle, lave à l'eau salée, sèche, évapore le solvant sous pression réduite, chromatographie le résidu sur silice (éluant : CH₂Cl₂-AcOEt 95-5). Ob obtient 1,31 g de dérivé silylé. On agite à température ambiante pendant 40 heures 13,06 g de dérivé silylé préparé comme indiqué ci-dessus dans 260 ml de diméthylformamide avec 45 g de dichromate de pyridinium, concentre partiellement sous pression réduite, verse dans 400 ml d'acide chlorhydrique (N) glacé, extrait à l'acétate d'éthyle, lave avec une solution de bicarbonate de sodium, sèche, évapore le solvant sous pression réduite, obtient 13,1 g d'acide brut. On agite 70 heures à température ambiante 12,53 g de l'acide obtenu ci-dessus dans 125 ml d'éthanol 100° en présence de 10 ml de chlorotriméthylsilane puis évapore les solvants sous pression réduite. Après chromatographie sur silice (éluant CH₂Cl₂-AcOEt 95-5 à 80-20), on obtient 3,40 g d'ester débloqué.

### Formation du tosylate.

On refroidit à 0°/+5°C 3,40 g du dérivé précédent dans 10 ml de pyridine, ajoute en 50 minutes 5,22 g de chlorure de tosyle dans 25 ml de dichlorométhane et agite 20 heures à température ambiante. On verse le milieu réactionnel dans 200 ml d'acide chlorhydrique N, extrait au dichlorométhane, lave à l'eau, sèche, évapore le solvant sous pression réduite, chromatographie le résidu sur silice (éluant : cyclohexane/AcOEt 2-8) et obtient 5,50 g de tosylate attendu.

### 3) Préparation du 1-((3-tosyloxyméthyl) cyclohexyl) carboxylate d'éthyle utilisé au départ de l'exemple 15.

### - formation du chlorure d'acide

On agite 3 heures au reflux 5 g d'acide 1,3-cyclohexane dicarboxylique dans 10 ml de chlorure de thionyle, distille l'excès de réactif, reprend par du toluène, évapore le solvant et récupère 6,04 g de produit attendu.

### - formation de l'ester

On refroidit à 0°C 1,69 ml d'éthanol et 50 ml de tétrahydrofuranne, ajoute en 30 minutes 18,12 ml de n-butyllithium en solution dans du tétrahydrofuranne (1,6 M/l) et agite 30 minutes à 0°/+5°C. On ajoute cette solution en 30 minutes à 6,04 g du chlorure d'acide obtenu précédemment dans 60 ml de tétrahydrofuranne à une température inférieure à 10°C.

### - réduction en alcool

En maintenant cette température, on ajoute en 30 minutes 58 ml de lithium tri(terbutoxyalumino hydrure), agite 1 heure, verse dans l'acide chlorhydrique N, extrait à l'acétate d'éthyle, lave à l'eau salée, sèche, évapore sous pression réduite, chromatographie le résidu (éluant : CH₂Cl₂/AcOEt 9-1) et obtient 1,37 g d'alcool.

### - formation du tosylate

On refroidit à 0°/+5°C 1,33 g de l'alcool précédent dans 5 ml de pyridine, ajoute en 20 minutes 1,63 g de chlorure de tosyle dans 5 ml de dichlorométhane et agite 20 heures à température ambiante. On verse le milieu réactionnel dans 100 ml d'acide chlorhydrique N, extrait à l'acétate d'éthyle, lave à l'eau, sèche, évapore le solvant sous pression réduite, chromatographie le résidu sur silice (éluant : CH₂Cl₂) et obtient 1,97 g de tosylate attendu.

### 4) Préparation du 1-((2-tosyloxyméthyl) cyclohexyl) carboxylate d'éthyle utilisé au départ de l'exemple 16.

### - formation de l'ester

On ajoute en 1 heure à température ambiante 2,23 ml de diazoacétate d'éthyle en solution dans 16 ml de dichlorométhane à 3,06 g de cis 1,2-cyclohexane diméthanol dans 100 ml de dichlorométhane en présence de quelques gouttes de borotrifluorure d'éthyl éthérate. On maintient 16 heures à température ambiante sous agitation, lave le milieu réactionnel à l'eau, sèche, évapore le solvant sous pression réduite, chromatographie le résidu sur silice (éluant : AcOEt/cyclohexane 20-80) et obtient 1,98 g de produit attendu.

### - formation du tosylate

On refroidit à 0°/+5° C 1,01 g de produit précédent dans 5 ml de pyridine, ajoute en 30 minutes 1,00 g de chlorure de tosyle dans 5 ml de dichlorométhane et agite 16 heures à température ambiante. On verse le milieu réactionnel dans 100 ml d'acide chlorhydrique N, extrait au dichlorométhane, lave à l'eau, sèche, évapore le solvant sous pression réduite, chromatographie le résidu sur silice (éluant : cyclohexane/AcOEt 2-8) et obtient 1,38 g de tosylate attendu.

### EXEMPLE 17 : Acide trans 4-((4-(carboxyméthyl) cyclohexyl) thio) 1-((6-chloro 1,3-benzodioxol-5-yl) méthyl) 2-(1,3-dioxolan-2-yl) 1H-imidazole 5-carboxylique.

### Stade A : amino (cyano) acétate d'éthyle.

On met en suspension 20 g de cyano (hydroxyimino) acétate d'éthyle dans 200 ml d'eau. On ajoute en 15 minutes à température ambiante, une solution saturée de bicarbonate de sodium préparée avec 16 g de NaHCO₃ et 160 ml d'eau. on chauffe le milieu réactionnel à 35°C, ajoute 68 g de dithionite de sodium, en 15 minutes, laisse revenir à température ambiante. On sature le milieu avec NaCl. On extrait avec 5 fois 500 ml de CH₂Cl₂ les phases organiques séchées et concentrées sous pression réduite et obteint 12,75 g de produit attendu.

### Stade B : ((acétyloxyacétyl) amino) cyano acétate d'éthyle.

On ajoute 24,3 g de chlorure d'acétoxyacétyle en solution dans 60 ml de tétrahydrofuranne à 23 g de produit obtenu comme au stade A dans 10 ml de tétrahydrofuranne en présence de 10 ml de pyridine. On agite à température ambiante pendant 4 heures, évapore le solvant, reprend à l'acétate d'éthyle, lave à l'eau salée, sèche, évapore le solvant sous pression réduite, cristallise dans l'éther éthylique et obtient 27,59 g de produit attendu. F = 82°C.

### Stade C : 3-((2-((acétyloxyacétyl) amino) 3-(amino (4-méthoxyphényl) méthyl) thio) 2-propénoate d'éthyle.

On ajoute goutte à goutte à 33,4 ml de 4-méthoxy benzyl mercaptan à 27,59 g du produit obtenu au stade B en solution dans 500 ml d'éthanol et en présence de 5 ml de triéthylamine. On agite 16 heures à température ambiante, évapore le solvant sous pression réduite, cristallise dans l'éther éthylique, essore et sèche sous pression réduite. On obtient 33,7 g de produit attendu. F = 145°C.

### Stade D : 2-((acétyloxy) méthyl) 5-(((4-méthoxyphényl) méthyl) thio) 1H-imidazole 4-carboxylate d'éthyle.

On chauffe au reflux 2,3 g de produit obtenu au stade C dans 50 ml d'acétate d'éthyle, ajoute 3,16 ml d'anhydride propane 1-phosphonique à 50% dans l'acétate d'éthyle, agite 30 minutes, laisse refroidir et neutralise avec une solution aqueuse saturée en bicarbonate de sodium. On extrait à l'acétate d'éthyle, lave à l'eau salée, sèche, évapore le solvant et obtient après empâtage dans l'éther et séchage, 2,09 g de produit attendu. F = 126°C.

### Stade E : 2-(hydroxyméthyl) 5-(((4-méthoxyphényl) méthyl) thio) 1H-imidazole 4-carboxylate d'éthyle.

On chauffe au reflux pendant 5 jours 26 g de produit obtenu comme au stade D dans 600 ml d'éthanol en présence de 1,3 g d'acide paratoluène sulfonique. On évapore le solvant sous pression réduite, reprend le résidu dans l'éthanol et obtient 6 g de produit attendu. Après concentration des liqueurs mères et chromatographie sur silice (éluant : AcOEt-cyclohexane 5-5), on récupère 9,44 g de produit attendu supplémentaire.

### Stade F : 2-formyl 5-(((4-méthoxyphényl) méthyl) thio) 1H-imidazole 4-carboxylate d'éthyle.

On agite 1 heure à température ambiante 8,6 g de produit obtenu au stade 6 dans 300 ml de dichlorométhane et 100 ml de méthanol en présence de 43 g de manganèse. On filtre, évapore le solvant de la solution obtenue, sous pression réduite et obtient 6 g de produit attendu utilisé tel quel pour le stade suivant.

### Stade G : 1-((6-chloro 1,3-benzodioxol-5-yl) méthyl) 2-formyl 4-(((4-méthoxyphényl) méthyl) thio) 1H-imidazole 5-carboxylate d'éthyle.

On agite à température ambiante pendant 50 heures 3 g de produit obtenu au stade F dans 150 ml de diméthylformamide avec 2,58 g de carbonate de potassium et 2,87 g de chlorure de 6-chloropipéronyle. On ajoute de nouveau 1,5 g de réactif chloré, agite 48 heures. On évapore le solvant sous pression réduite, reprend le résidu par 150 ml d'une solution aqueuse saturée en chlorure d'ammonium, extrait à l'acétate d'éthyle, lave à l'eau salée, sèche, évapore le solvant, chromatographie le résidu sur silice (éluant : AcOEt-cyclohexane 2-8) et obtient après empâtage dans l'éther ispropylique 3,71 g de produit attendu.

### Stade H : bis ((1-((6-chloro 1,3-benzodioxol-5-yl) méthyl) 2-formyl 5-éthoxycarbonyl 1H-imidazol-4-yl) thiolate) de mercure.

On refroidit à 0°C une suspension comprenant 2,6 g de produit obtenu comme au stade G dans 2,6 ml d'anisole et 13 ml d'acide trifluoroacétique. On ajoute 1,13 g de trifluoroacétate de mercure, laisse revenir à température ambiante, agite 2 heures, évapore le solvant sous pression réduite, reprend le résidu dans l'éthanol, essore et sèche le sel de mercure obtenu. On récupère 1,83 g de produit attendu.

### Stade I : bis ((1-((6-chloro 1,3-benzodioxol-5-yl) méthyl) 2-(1,3-dioxolan-2-yl) 5-éthoxycarbonyl 1H-imidazol-4-yl) thiolate) de mercure.

On chauffe au reflux 500 mg de produit obtenu au stade H dans 100 ml de toluène avec 165 mg d'éthylène glycol et 25 mg d'acide paratoluène sulfonique, en éliminant l'eau formée. On évapore le solvant sous pression réduite, essore et sèche les cristaux formés. Après concentration des liqueurs mères et empâtage dans l'éther éthylique, on essore et sèche le produit cristallisé supplémentaire. On obtient 540 mg de produit attendu. F = 186°C.

### Stade J : 1-((6-chloro 1,3-benzodioxol-5-yl) méthyl) 2-(1,3-dioxolan-2-yl) 4-mercapto 1H-imidazole 5-carboxylate d'éthyle.

On fait barbotter pendant 10 minutes un courant de sulfure d'hydrogène dans un mélange comprenant 530 mg de produit obtenu au stade I dans 100 ml d'acétate d'éthyle, filtre, lave à l'acétate d'éthyle, concentre sous pression réduite et obtient 500 mg de produit attendu utilisé tel quel pour le stade suivant.

### Stade K : trans 1-((6-chloro 1,3-benzodioxol-5-yl) méthyl) 2-(1,3-dioxolan-2-yl) 4-(4-(((éthoxycarbonyl) méthyl) cyclohexyl) thio) 1H-imidazole 5-carboxylate d'éthyle.

On ajoute 60 mg d'hydrure de sodium à 50% dans l'huile à 500 mg du produit obtenu au stade J en solution dans 20 ml de N,N-diméthylformamide, agite pendant 15 minutes, ajoute 610 mg de 4-iodo cyclohexane acétate d'éthyle et maintient 16 heures sous agitation. On ajoute 50 ml d'une solution aqueuse saturée en chlorure d'ammonium, extrait au dichlorométhane, lave la phase organique à l'eau salée, évapore le solvant sous pression réduite et chromatographie le résidu sur silice (éluant : AcOEt-cyclohexane 3-7). On obtient 380 mg de produit attendu.

### Stade L : Acide trans 4-((4-(carboxyméthyl) cyclohexyl) thio) 1-((6-chloro 1,3-benzodioxol-5-yl) méthyl) 2-(1,3-dioxolan-2-yl) 1H-imidazole 5-carboxylique.

On mélange 380 mg de produit obtenu au stade K, 20 ml de soude 2N et 20 ml d'éthanol, ajoute du tétrahydrofuranne jusqu'à dissolution et maintient 2 jours sous agitation à température ambiante. On évapore les solvant sous pression réduite, reprend le résidu par de l'eau, acidifie à pH = 1 pr de l'acide chlorhydrique 2N, filtre, lave à l'eau, sèche sous pression réduite à 60°C et recueille 280 mg de produit attendu. F = 171°C.

### Préparation du 4-iodo cyclohexane acétate d'éthyle utilisé au stade K de l'exemple 17.

### Stade a : trans 4-iodo cyclohexanol.

On verse goutte à goutte 44 g de 7-oxabicyclo [2.2.1] heptane dans 250 ml d'acide iodhydrique à 57% en maitenant à température ambiante et agite pendant 4 heures. On verse le milieu réactionnel dans l'eau glacée, extrait à l'éther éthylique, lave les phases éthérées avec 200 ml de thiosulfate de sodium 0,5N , lave à l'eau salée, sèche et évapore les solvants. On obtient 96,47 g de produit attendu.
F = 53,4°C.

### Stade b : 4-iodo cyclohexanone.

On ajoute 52,4 g de permanganate de potassium à 50 g de produit obtenu au stade a) en solution dans 500 ml d'acétonitrile. On agite 16 heures à température ambiante, filtre, évapore le solvant sous pression réduite, chromatographie le résidu sur silice (éluant : AcOEt -cyclohexane 20-80) et obtient 36,17 g de produit attendu. F = 48,7°C.

### Stade c : 4-iodo cyclohexylidène acétate d'éthyle.

On ajoute lentement 6,6 g d'hydrure de sodium à 60% dans l'huile puis 36,04 g du produit obtenu au stade b) dissous dans 100 ml de tétrahydrofuranne à un mélange comprenant 32 ml de triéthylphosphonoacétate dans 200 ml de tétrahydrofuranne. On maintient sous agitation à température ambiante, ajoute à deux reprises 2 g d'hydrure de sodium puis verse le milieu réactionnel dans l'eau, neutralise par addition de 150 ml d'une solution aqueuse saturée en chlorure d'ammonium. On extrait à l'acétate d'éthyle, lave à l'eau salée, sèche, évapore les solvants sous pression réduite, purifie par chromatographie sur silice (éluant : AcOEt-cyclohexane 3-7) et obtient 38,25 g de produit attendu.

### Stade d : 4-iodo cyclohexane acétate d'éthyle.

On hydrogène sous 600 mbars de pression pendant 48 heures un mélange comprenant 38,25 g de produit obtenu au stade c), 300 ml de tétrahydrofuranne et 1,5 g d'oxyde de platine puis 5 g de platine à 5% sur charbon actif. On filtre, évapore le solvant sous pression réduite, chromatographie le résidu sur silice (éluant AcOEt-cyclohexane 10-90) et obtient 27,01 g de produit attendu.

En opérant comme à l'exemple 17 en omettant les stades E, F et I, en utilisant au départ le produit obtenu à l'exemple 17 stade A et le chlorure d'acide approprié, on a préparé les produits suivants :

### EXEMPLE 18 : Acide trans 4-((4-(carboxyméthyl) cyclohexyl) thio) 1-((6-chloro 1,3-benzodioxol-5-yl) méthyl) 2-(2-furanyl) 1H-imidazole 5-carboxylique.

F = 185°C.

### EXEMPLE 19 : Acide trans 4-((4-(carboxyméthyl) cyclohexyl) thio) 1-((6(-chloro 1,3-benzodioxol-5-yl) méthyl) 2-cyclopentyl 1H-imidazole 5-carboxylique.

F = 171°C.

### EXEMPLE 20 de réference: Acide trans 3-((4-(carboxyméthyl) cyclohexyl) thio) 1-((6-chloro 1,3-benzodioxol-5-yl) méthyl) 2-(2-thiényl) 1H-imidazole 5-carboxylique.

F = 195°C.

### EXEMPLE 21 de référence: Acide trans (3-((4-(carboxyméthyl) cyclohexyl) thio) 1-((6-chloro 1,3-benzodioxol-5-yl) méthyl) 2-cyclohexyl 1H-imidazole 5-carboxylique.

F = 165°C.

On opère comme à l'exemple 17 en utilisant à la place du dérivé iodé le 4-bromo cyclohexyl carboxylate d'éthyle comme à l'exemple 13 pour obtenir le produit de l'exemple 22.

### EXEMPLE 22 : Acide 4-((4-(carboxycyclohexyl) thio) 1-((6-chloro 1,3-benzodioxol-5-yl) méthyl 2-(2-furanyl) 1H-imidazole 5-carboxylique (cis/trans).

F = 165°C.

### EXEMPLE 23 : Sel de disodium de l'acide 4-((4-(carboxyméthyl) cyclohexyl) thio) 1-((6-chloro 1,3-benzodioxol-5-yl) méthyl) 2-propyl 1H-imidazole 5-carboxylique (cis).

On recristallise 1 g d'acide 4-((4-(carboxyméthyl) cyclohexyl) thio) 1-((6-chloro 1,3-benzodioxol-5-yl) méthyl) 2-propyl 1H-imidazole 5-carboxylique préparé comme à l'exemple 10, dans l'éthanol et recueille 780 mg de dérivé cis pur que l'on dissout dans 31,51 ml de soude 0,1N. On évapore les solvants sous pression réduite et récupère 949 mg de disel de sodium cis attendu. F = 166°C.

### EXEMPLE 24 : Sel de dipotassium de l'acide 4-((4-(carboxyméthyl) cyclohexyl) thio) 1-((6-chloro 1,3-benzodioxol-5-yl) méthyl) 2-propyl 1H-imidazole 5-carboxylique.

On opère comme indiqué à l'exemple 23 en remplaçant la soude 0,1N par de la potasse 0,1N. On obtient le disel de potassium attendu. F > 250°C.

### EXEMPLE 25 : COMPOSITION PHARMACEUTIQUE :

On a préparé des comprimés répondant à la formule suivante :

| | |
|---|---|
| Produit de l'exemple 2 | 50 mg |
| Excipient pour un comprimé terminé à (détail de l'excipient : lactose, talc, amidon, stéarate de magnésium). | 200 mg |

### RESULTATS PHARMACOLOGIQUES

### 1) ETUDE DE L'AFFINITE POUR LE RECEPTEUR A DE L'ENDOTHELINE

On effectue une préparation membranaire à partir de coeur (ventricules) de rat. Le tissu est broyé au POLYTRON dans un tampon Tris 50 mM pH = 7,4.

Après 30 minutes à 25 °C (B.M.) l'homogénat est centrifugé à 30000 g pendant 15 minutes (2 centrifugations avec reprise intermediaire dans le tampon Tris pH. 7,4).

Les culots sont remis en suspension dans un tampon d'incubation (Tris 25 mM, pepstatine A 5 microg/ml, aprotinine 3 microg/ml, PMSF 0,1 mM, EDTA 3mM, EGTA 1mM pH 7,4).

On répartit des fractions aliquotes de 2 ml dans des tubes à hémolyse et ajoute de la 125I Endothéline (environ 50000 dpm/tube) et le produit à étudier. (Le produit est d'abord testé à 3*10⁻⁵ M en triple). Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %.

La liaison non spécifique est déterminée par addition d'endothéline à 10⁻⁶ M (en triple). On incube à 25 °C pendant 60 minutes, remet au bain-marie à 0 °C, pendant 5 minutes, filtre sous pression réduite, rince au tampon Tris 7,4 et compte la radioactivité en présence du scintillant Triton.

Le résultat est exprimé directement en concentration inhibitrice 50 % (CI50), c'est-à-dire en concentration de produit étudié exprimée en nM, nécessaire pour déplacer 50 % de la radioactivité spécifique fixée sur le récepteur étudié.

### Résultat :

Les CI50 trouvées pour les produits des exemples sont données dans le tableau I ci-après, en nanomoles.

**TABLEAU I**

| Produit des exemples | Récepteur A de l'endothéline CI50 en nanomoles |
|---|---|
| 2 | 1,1 |
| 6 (réference) | 1,2 |
| 8 (réference) | 1,9 |
| 10 | 1,2 |
| 12 (réference) | 1,1 |

### 2) ETUDE DE L'AFFINITE POUR LE RECEPTEUR B DE L'ENDOTHELINE

On effectue une préparation membranaire à partir de cortex postérieur plus cervelet de rat. Le tissu est broyé au POLYTRON dans un tampon Tris 50 mM pH = 7,4.

Après 30 minutes à 25 °C (B.M.) l'homogénat est centrifugé à 30000 g pendant 15 minutes (2 centrifugations avec reprise intermediaire dans le tampon Tris pH. 7,4).

Les culots sont remis en suspension dans un tampon d'incubation (Tris 25 mM, pepstatine A 5 microg/ml, aprotinine 3 microg/ml, PMSF 0,1 mM, EDTA 3mM, EGTA 1mM pH 7,4).

On répartit des fractions aliquotes de 2 ml dans des tubes à hémolyse et ajoute de la 125I Endothéline (environ 50000 dpm/tube) et le produit à étudier. (Le produit est d'abord testé à 3*10⁻⁵ M en triple). Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %.

La liaison non spécifique est déterminée par addition d'endothéline à 10⁻⁶ M (en triple). On incube à 25 °C pendant 60 minutes, remet au bain-marie à 0 °C, pendant 5 minutes, filtre sous pression réduite, rince au tampon Tris 7,4 et compte la radioactivité en présence du scintillant Triton.

Le résultat est exprimé directement en concentration inhibitrice 50 % (CI50), c'est-à-dire en concentration de produit étudié exprimée en nM, nécessaire pour déplacer 50 % de la radioactivité spécifique fixée sur le récepteur étudié.

### Résultats :

Les CI50 trouvées pour les produits des exemples sont données dans le tableau I ci-après, en nanomoles.

**TABLEAU I**

| Produit des exemples | Récepteur B de l'endothéline CI₅₀ en nanomoles |
|---|---|
| 2 | 194 |
| 6 (réference) | 293 |
| 8 (réference) | 600 |
| 10 (réference) | 210 |

### 3) Test d'activité antagoniste de l'endothéline chez le rat démédullé

Des rats mâles Sprague-Dawley (250 à 350 g) sont anesthésiés (pentobarbital sodique 60 mg/kg injecté par voie intrapéritonale). L'animal est placé sous respiration assistée, une section bilatérale des nerfs vagues est effectuée. Le rat est ensuite démédullé.

La pression artérielle moyenne est enregistrée grâce à un cathéter (PE50) hépariné placé dans la carotide de l'animal et relié à un enregistreur par l'intermédiaire d'un capteur de pression et d'un amplificateur (pressure processor Gould). Un cathéter est implanté dans la veine pudendale afin de permettre l'injection des molécules à étudier. Après une période de stabilisation (15 mn environ), le produit ou le solvant est injecté 10 minutes avant une gamme de doses croissantes d'endothéline injectées toutes les 2 minutes (0.1-0.3-1-3-10 µg/kg).

L'activité antagoniste des produits est estimée par le pourcentage d'inhibition de l'augmentation de pression induite par l'endothéline aux doses de 3 et 10 µg/kg.

### Résultats

| EXEMPLES | Doses mg/kg | % inhibition de vasoconstriction | |
|---|---|---|---|
| | | ET₁ 3 µg/kg | ET₁ 10 µg/kg |
| 2 | 0.3 | -56 | -39 |
| | 1.0 | -60 | -65 |
| | 3.0 | -63 | -77 |
| 3 | 1.0 | -52 | -53 |
| | 3.0 | -64 | -71 |
| 4 (réf.) | 10.0 | -49 | -38 |
| 6 | 1.0 | -42 | -33 |
| (réference) | 3.0 | -49 | -37 |
| 8 | 1.0 | -40 | -22 |
| (réference) | 3.0 | -57 | -47 |
| 10 | 1.0 | -59 | -39 |
| (réference) | 3.0 | -58 | -61 |
| 12 (réf.) | 10.0 | -30 | -35 |

### 4) Mise en évidence de l'activité antagoniste de l'endothéline, sur aorte isolée de rat

L'aorte thoracique est prélevée sur des rats mâles Wistar (350 g environ) (IFFA CREDO France) anesthésiés au pentobarbital (60 mg/kg IP) puis exsanguinés. L'aorte est mise rapidement dans une solution physiologique à température ambiante. Un anneau sans endothélium de 1 à 2 mm est monté dans un bain à organe isolé contenant 5 ml d'une solution physiologique suivante (composition en mM NaCl : 118.4 ; KCl : 4.7 ; MgSO₄, 7 H₂O : 1.2 ; CaCl₂, 2 H₂O : 2.5 ; KH₂PO₄ : 1.2 ; NaHCO₃ : 25 ; glucose : 10.1) le milieu est maintenu à 37°C et oxygéné par un mélange oxygène (95 %) gaz carbonique (5 %). La tension initiale imposée est de 2 g, les anneaux sont laissés au repos pendant 60 à 90 minutes. La contraction de référence est provoquée par l'ajout de 2 concentrations successives de 3 µM de noradrénaline, laissées en contact 15 minutes, suivi de 3 lavages, la seconde réponse sert de contraction de référence. Après retour à la ligne de base, on incube pendant 15 minutes avec une concentration du produit à tester ou le solvant puis des concentrations cumulées d'endothéline sont additionnées toutes les 5 minutes (concentrations de 1 à 1000 µM). L'inhibition induite par le produit est calculée en % de diminution par rapport à la réactivité des courbes témoins en 2 points : concentration de 10 et 30 nM d'endothéline.

### Résultats

| EXEMPLES | Concentration d'antagoniste µM | % inhibition de contraction | |
|---|---|---|---|
| | | ET 10 nM | ET 30 nM |
| 2 | 0.1 | -71 | -61 |
| | 1.0 | -94 | -100 |
| 6 (réf.) | 0.1 | -96 | -99 |
| | 1.0 | -96 | -98 |
| 8 (réf.) | 0.1 | -97 | -97 |
| | 1.0 | -95 | -96 |
| 12 (réf.) | 0.1 | -88 | -65 |
| | 1.0 | -98 | -98 |

### 5) Protection contre la mort subite induite par injection IV d'endothéline (ET1) chez la souris femelle

Une dose léthale d'ET1 (10 nmole/kg IV) est injectée chez des souris femelles. Le solvant ou les molécules à étudier sont injectés 10 minutes avant (voie i.v.) ou 30 minutes avant (voie orale) l'endothéline.

La protection est calculée sous forme de DE₅₀ (dose efficace 50 %, ou dose capable de diminuer la mortalité de 50 % par rapport au groupe témoin).

### Résultats

| EXEMPLES | DE₅₀ mg/kg | |
|---|---|---|
| | IV | PO |
| 2 | 0,8 | 1,1 |
| 3 | 5,0 | 24,1 |
| 6 (réf.) | 3,9 | NT |
| 8 (réf.) | 3,5 | NT |
| 10 (réf.) | 3,3 | 3,2 |

| | | |
|---|---|---|
| NT signifie "non testé" | | |

## Revendications

1. Produits sélectionnés dans le groupe consistant en les produits dont les noms suivent :
- acide 4-((4-(carboxyméthylène) cyclohexyl) thio)-1-((6-chloro-1,3-benzodioxol-5-yl) méthyl)-2-propyl-1H-imidazole-5-carboxylique,
- acide 4-((5-carboxypentyl) thio)-1-((6-chloro-1,3-benzodioxol-5-yl) méthyl)-2-(1,3-dioxolan-2-yl)-1H-imidazole-5-carboxylique,
- acide 4-((4-(carboxyméthyl) cyclohexyl) thio)-1-((6-chloro-1,3-benzodioxol-5-yl) méthyl)-2-propyl-1H-imidazole-5-carboxylique,
- acide 1-((6-chloro 1,3-benzodioxol-5-yl) méthyl) 4-((4-carboxycyclohexyl) thio) 2-propyl 1H-imidazole 5-carboxylique,
- acide 4(((4-carboxycyclohexyl) méthyl) thio) 1-((6-chloro 1,3-benzodioxol-5-yl) méthyl) 2-propyl 1H-imidazole 5-carboxylique,
- acide trans 4-((4-(carboxyméthyl) cyclohexyl) thio) 1-((6-chloro 1,3-benzodioxol-5-yl) méthyl) 2-(1,3-dioxolan-2-yl) 1H-imidazole 5-carboxylique,
- acide 4-((4-(carboxyméthyl) cyclohexyl) thio) 1-((6-chloro 1,3-benzodioxol-5-yl) méthyl) 2-(2-furanyl) 1H-imidazole 5-carboxylique,
- acide trans 4-((4-(carboxyméthyl) cyclohexyl) thio) 1-((6-chloro 1,3-benzodioxol-5-yl) méthyl) 2-cyclopentyl 1H-imidazole 5-carboxylique,
- acide 4-((4-carboxycyclohexyl) thio) 1-((6-chloro 1,3-benzodioxol-5-yl) méthyl) 2-(2-furanyl) 1H-imidazole 5-carboxylique,
- sel de disodium de l'acide 4-((4-(carboxyméthyl) cyclohexyl) thio) 1-((6-chloro 1,3-benzodioxol-5-yl) méthyl) 2-propyl 1H-imidazole 5-carboxylique,
- sel de dipotassium de l'acide 4-((4-(carboxyméthyl) cyclohexyl) thio) 1-((6-chloro 1,3-benzodioxol-5-yl) méthyl) 2-propyl 1H-imidazole 5-carboxylique,

2. A titre de médicaments, les produits tels que définis à la revendication 1, ainsi que les sels d'addition avec les acides minéraux et organiques ou avec les bases minérales et organiques pharmaceutiquement acceptables desdits produits.

3. Les compositions pharmaceutiques contenant à titre de principe actif, l'un au moins des médicaments tels que définis à la revendication 2.

4. Utilisation des produits tels que définis à la revendication 1, pour la préparation de compositions pharmaceutiques destinées au traitement d'affections résultant d'une stimulation anormale des récepteurs de l'endothéline et notamment destinées au traitement de l'hypertension induite par l'endothéline, de tous les spasmes vasculaires, au traitement des suites d'une hémorragie cérébrale, des insuffisances rénales, de l'infarctus du myocarde, de l'insuffisance cardiaque, à la prévention des resténoses post-angioplastie ainsi que des fibroses cardiaques et vasculaires.

## Claims

1. Products selected from the group consisting of the products having the following names:
- 4-((4-(carboxymethylene)cyclohexyl)thio)-1-((6-chloro-1,3-benzodioxol-5-yl)methyl)-2-propyl-1H-imidazole-5-carboxylic acid,
- 4-((5-carboxypentyl)thio)-1-((6-chloro-1,3-benzodioxol-5-yl)methyl)-2-(1,3-dioxolan-2-yl)-1H-imidazole-5-carboxylic acid,
- 4-((4-(carboxymethyl)cyclohexyl)thio)-1-((6-chloro-1,3-benzodioxol-5-yl)methyl)-2-propyl-1H-imidazole-5-carboxylic acid,
- 1-((6-chloro-1,3-benzodioxol-5-yl)methyl)-4-((4-carboxycyclohexyl)thio)-2-propyl-1H-imidazole-5-carboxylic acid,
- 4-(((4-carboxycyclohexyl)methyl)thio)-1-((6-chloro-1,3-benzodioxol-5-yl)methyl)-2-propyl-1H-imidazole-5-carboxylic acid,
- trans-4-((4-(carboxymethyl)cyclohexyl)thio)-1-((6-chloro-1,3-benzodioxol-5-yl)methyl)-2-(1,3-dioxolan-2-yl)-1H-imidazole-5-carboxylic acid,
- 4-((4-(carboxymethyl)cyclohexyl)thio)-1-((6-chloro-1,3-benzodioxol-5-yl)methyl)-2-(2-furyl)-1H-imidazole-5-carboxylic acid,
- trans-4-((4-(carboxymethyl)cyclohexyl)thio)-1-((6-chloro-1,3-benzodioxol-5-yl)methyl)-2-cyclopentyl-1H-imidazole-5-carboxylic acid,
- 4-((4-carboxycyclohexyl)thio)-1-((6-chloro-1,3-benzodioxol-5-yl)methyl)-2-(2-furyl)-1H-imidazole-5-carboxylic acid,
- disodium salt of 4-((4-(carboxymethyl)cyclohexyl)-thio)-1-((6-chloro-1,3-benzodioxol-5-yl)methyl)-2-propyl-1H-imidazole-5-carboxylic acid,
- dipotassium salt of 4-((4-(carboxymethyl)cyclohexyl)-thio)-1-((6-chloro-1,3-benzodioxol-5-yl)methyl)-2-propyl-1H-imidazole-5-carboxylic acid.

2. As medicaments, the products as defined in Claim 1 and the addition salts of the said products with pharmaceutically acceptable inorganic and organic acids or with pharmaceutically acceptable inorganic and organic bases.

3. Pharmaceutical compositions comprising, as active principle, at least one of the medicaments as defined in Claim 2.

4. Use of the products as defined in Claim 1 in the preparation of pharmaceutical compositions intended for the treatment of conditions resulting from abnormal stimulation of endothelin receptors and intended in particular for the treatment of endothelin-induced hypertension, of all vascular spasms, of the effects of a cerebral haemorrhage, of renal insufficiency, of myocardial infarction or of cardiac insufficiency, in the prevention of post-angioplastic restenosis, and cardiac and vascular fibrosis.

## Patentansprüche

1. Produkte, ausgewählt aus der Gruppe, die aus den Produkten besteht, deren Namen folgen:
- 4-((4-(Carboxymethylen)cyclohexyl)thio)-1-((6-chlor-1,3-benzodioxol-5-yl)methyl)-2-propyl-1H-imidazol-5-carbonsäure,
- 4-((5-Carboxypentyl)thio)-1-((6-chlor-1,3-benzodioxol-5-yl)methyl)-2-(1,3-dioxolan-2-yl)-1H-imidazol-5-carbonsäure,
- 4-((4-(Carboxymethyl)cyclohexyl)thio)-1-((6-chlor-1,3-benzodioxol-5-yl)methyl)-2-propyl-1H-imidazol-5-carbonsäure,
- 1-((6-Chlor-1,3-benzodioxol-5-yl)methyl)-4-((4-carboxycyclohexyl)thio)-2-propyl-1H-imidazol-5-carbonsäure,
- 4-(((4-Carboxycyclohexyl)methyl)thio)-1-((6-chlor-1,3-benzodioxol-5-yl)methyl)-2-propyl-1H-imidazol-5-carbonsäure,
- trans-4-((4-(Carboxymethyl)cyclohexyl)thio)-1-((6-chlor-1,3-benzodioxol-5-yl)-methyl)-2-(1,3-dioxolan-2-yl)-1H-imidazol-5-carbonsäure,
- 4-((4-(Carboxymethyl)cyclohexyl)thio)-1-((6-chlor-1,3-benzodioxol-5-yl)methyl)-2-(2-furanyl)-1H-imidazol-5-carbonsäure,
- trans-4-((4-(Carboxymethyl)cyclohexyl)thio)-1-((6-chlor-1,3-benzodioxol-5-yl)-methyl)-2-cyclopentyl-1H-imidazol-5-carbonsäure,
- 4-((4-Carboxycyclohexyl)thio)-1-((6-chlor-1,3-benzodioxol-5-yl)methyl)-2-(2-furanyl)-1H-imidazol-5-carbonsäure,
- 4-((4-(Carboxymethyl)cyclohexyl)thio)-1-((6-chlor-1,3-benzodioxol-5-yl)methyl)-2-propyl-1H-imidazol-5-carbonsäure-Dinatriumsalz,
- 4-((4-(Carboxymethyl)cyclohexyl)thio)-1-((6-chlor-1,3-benzodioxol-5-yl)methyl)-2-propyl-1H-imidazol-5-carbonsäure-Dikaliumsalz.

2. Die wie in Anspruch 1 definierten Produkte sowie die Additionssalze besagter Produkte mit den anorganischen und organischen Säuren oder mit den anorganischen und organischen Basen, die pharmazeutisch annehmbar sind, als Medikamente.

3. Die pharmazeutischen Zusammensetzungen, die als Wirkstoff wenigstens eines der wie in Anspruch 2 definierten Medikamente enthalten.

4. Verwendung der wie in Anspruch 1 definierten Produkte zur Herstellung von pharmazeutischen Zusammensetzungen, die für die Behandlung von Erkrankungen, die aus einer anormalen Stimulierung der Endothelinrezeptoren resultieren, bestimmt sind und insbesondere für die Behandlung des durch Endothelin ausgelösten Bluthochdrucks, aller Gefäßspasmen, für die Behandlung der Folgen einer Hirnblutung, der Niereninsuffizienzen, des Myokardinfarkts, der Herzinsuffizienz, für die Prävention der Restenosen nach Angioplastie sowie der Herz- und Gefäßfibrosen bestimmt sind.
